# FASCICULE DE BREVET EUROPEEN

(11) **EP 2 343 374 B1**
(45) Date de publication et mention de la délivrance du brevet: **09.05.2012**
(21) Numéro de dépôt: 10005982.3
(22) Date de dépôt: 23.09.2002
(51) Int. Cl.: C12N 15/51, C07K 14/08, A61K 39/29, G01N 33/576, C12Q 1/70

(54) **Molécules d'acides nucléiques d'HDV, leurs fragments et leurs applications**
Moleküle von HDV-Nukleinsäuren, ihre Fragmente und Anwendungen
Molecules of HDV nucleic acids, fragments and applications thereof

(30) Priorité: 24.09.2001 FR 0112285
(43) Date de publication de la demande: 13.07.2011
(62) Demande divisionnaire de: 02781380.7
(73) Titulaire: Assistance Publique - Hôpitaux de Paris, 75004 Paris (FR)
(72) Inventeur: Deny, Paul, 78420 Carrières sur Seine (FR); Radjef, Nadjia, 94000 Créteil (FR); Huc-Anais, Patricia, 97150 Saint-Martin (FR)
(74) Mandataire: Rançon, Xavier Lucien Abel

(56) Documents cités:
- J.C. WU ET AL.: "Characterisation and phylogenetic analysis of a novel hepatitis D virus strain discovered by restriction fragment length polymorphism", J. GEN VIROL., vol. 79, 1998, pages 1105-1113, XP002196403,
- SAKUGAWA HIROSHI ET AL: "Hepatitis delta virus genotype IIb predominates in an endemic area, Okinawa, Japan", JOURNAL OF MEDICAL VIROLOGY, vol. 58, no. 4, août 1999 (1999-08), pages 366-372, XP002607358, ISSN: 0146-6615
- IMAZEKI ET AL.: "Heterogeneity and evolution rates of delta virus RNA sequences", J. VIROL., vol. 64, no. 11, 1990, pages 5594-5599, XP001073432,
- IVANIUSHINA V ET AL: "Hepatitis delta virus genotypes I and II cocirculate in an endemic area of Yakutia, Russia.", THE JOURNAL OF GENERAL VIROLOGY NOV 2001 LNKD- PUBMED:11602783, vol. 82, no. Pt 11, novembre 2001 (2001-11), pages 2709-2718, XP002607359, ISSN: 0022-1317

## Description

La présente invention est relative à des molécules d'acides nucléiques issues de nouvelles souches ou isolats du virus de l'hépatite D constituant des génotypes différents des génotypes connus I, II et III, à leurs fragments, aux protéines correspondantes, ainsi qu'à leurs applications, en tant que réactifs de diagnostic.

La présente invention est également relative à une méthode de diagnostic sensible du virus de l'hépatite D (ou virus de l'hépatite delta) ainsi qu'à une méthode de suivi épidémiologique des infections à HDV.

Le virus de l'hépatite D (VHD ou HDV, pour *hepatitis D virus*) ou delta est un virus satellite de l'hépatite B. Ce virus a une structure particulière : structure chimère associant aux composants spécifiques de l'HDV (ARN viral et protéines HD), une enveloppe comportant les trois glycoprotéines de l'HBV : grande (préS1-préS2-S), moyenne (préS2-S) et petite (S). Le diamètre moyen des particules HDV se situe entre celui des particules HBV matures (particules de DANE : 42 nm) et celui des enveloppes vides de l'HBV (formes sphériques ou filamenteuses : 22 nm) et la densité de flottaison est de 1,24-1,25 g/cm³.

Au sein des virions, l'ARN HDV est circulaire et de polarité négative. Plus petit génome connu des virus infectant les mammifères, ce brin monocaténaire circulaire fermé, a un pourcentage élevé de GC (60%).

L'ARN HDV se réplique indépendamment de l'HBV, dont le rôle se limite à fournir l'enveloppe de l'HDV. Les seules protéines retrouvées (sHD et LHD) sont codées par l'ARN antigénomique qui, dans la cellule infectée, est complet, circulaire et pseudo double-brin, sert d'intermédiaire de réplication et supporte l'édition.

L'ARN HDV appartient à un type de ribozyme spécifique. La réaction d'auto-clivage nécessite l'ARN et un cation divalent (Mg⁺⁺). Le clivage crée une extrémité 2',3' phosphate cyclique et une extrémité 5' hydroxyle.

Les ribozymes delta (génomique et anti-génomique) ont une configuration secondaire voisine en pseudo-noeud. Les séquences impliquées incluent principalement ou exclusivement des séquences situées en 3' du site d'auto-clivage (environ 84 nucléotides).

Au cours du cycle viral, l'ARNm de l'HDV code pour une protéine dont il existe deux formes : une protéine de 194-195 acides aminés (forme 's' pour small) de 24 kilodaltons (kDa) et une protéine de 214 acides aminés (forme 'L' pour Large) de 27 kDa, qui existent en proportions variables. Ces protéines portent l'antigénicité 'delta' et sont détectées dans le foie ou le sérum de patients ou d'animaux infectés (chimpanzé, marmotte). Ces deux protéines virales sHD et LHD s'initient au premier ATG du cadre de lecture ouvert situé à la position 1598 (selon la numérotation de Wang et al., 1986 ou 1987) de l'ARN antigénomique. Au cours de la réplication, une mutation, dépendante d'une enzyme cellulaire 'l'adénosine déaminase dépendante des ARN double brin' apparaît à la position 1012, transformant le codon stop ambre (UAG) en codon tryptophane (UGG), rallongeant en 3' le cadre de lecture de 19 ou 20 codons, et conférant aux deux formes sHD et LHD des propriétés différentes.

L'ARNm est terminé par une queue de poly(A), 15 nucléotides après le signal consensus de polyadénylation, AAUAAA (positions 954-959).

Dans le cycle de réplication, les fonctions des protéines de 24 et 27 Kd s'opposent : sHD active la réplication virale alors que LHD la réprime et joue un rôle dans l'assemblage des particules virales. Ces protéines sont phosphorylées sur les résidus sérine mais non glycosylées (Tableau I). Elles sont constituées de domaines fonctionnels communs et d'un domaine spécifique à la grande protéine LHD.

**Tableau I : Résumé et comparaison des fonctions des deux formes p24 et p27**

| Activités biochimiques et biologiques | p24 (S) | p27 (L) |
|---|---|---|
| Acides aminés | 195 | 214 |
| Transactivation de la réplication | + | - |
| Trans-inhibition de la réplication | - | + |
| Dimérisation et polymérisation | + | + |
| Fixation à l'ARN | + | + |
| Stabilisation de l'ARN | + | + |
| Localisation nucléaire | + | + |
| Assemblage | - | + |
| Phosphorylation | + | +(x6) |
| 19 aa carboxy-terminaux spécifiques | - | + |
| Farnésylation | - | + |
| | | |

Brièvement, les différents domaines de ces deux protéines sont les suivants :
* domaines communs
   - Le domaine de polymérisation,_qui comprend la séquence entre les résidus d'acides aminés 13 et 48 formée d'un agencement de leucine ou isoleucine, organisé en hélice α de type "leucine zipper" (crémaillère à leucine), impliquée dans la polymérisation des protéines, indispensable à (i) la *trans* activation de la réplication virale par l'Ag-sHD, (ii) l'inhibition de la réplication par l'Ag-LHD et (iii) l'assemblage des complexes sHD-LHD dans les enveloppes de l'HBV.
   - Le signal de localisation nucléaire (SLN) qui implique deux séquences de localisation nucléaire identifiées dans la région 67-88, essentielles pour transloquer l'Ag-sHD synthétisé dans le cytoplasme et, peut-être la ribonucléoprotéine après son entrée dans la cellule, vers le noyau.
   - Le site de fixation à l'ARN qui repose sur deux séquences riches en arginine localisées entre les résidus 97 et 163 qui permettent l'attachement des protéines sHD sur l'ARN génomique ou antigénomique. Cette fixation est indispensable pour que l'Ag-sHD active la réplication.
* domaines spécifiques

Les 19-20 acides aminés situés à l'extrémité COOH de la grande protéine ont un rôle important dans le cycle de l'HDV. En effet, ces acides aminés (aa 195-214), interviennent dans l'assemblage des particules virales (Chang et. al., 1991). Cette activité pourrait être en partie liée à la présence d'une cystéine à la position 211 (Glenn et al., 1992), conservée pour tous les génomes viraux caractérisés à ce jour. Cette cystéine, située 4 acides aminés avant l'extrémité COOH de la protéine, forme une boîte "CXXX" et fixe un groupement farnésyle (Glenn et al., 1992) chaîne de 15 carbones dérivée de l'acide mévalonique, par action d'une farnésyltransférase. Cette maturation post-traductionelle oriente les protéines vers les membranes cellulaires.

La petite et la grande protéine ont été par ailleurs différenciées par des anticorps monoclonaux (clone 9E4) (Hwang et Lai, 1993a). Ces anticorps ne reconnaissaient que sHD (Lai et. al., 1993). La séquence en acides aminés de la petite protéine étant incluse dans la grande, ces résultats suggèrent une conformation différente entre sHD et LHD au sein des 30 acides aminés carboxyterminaux de la petite protéine sHD, suggérant que l'épitope reconnu sur sHD soit masqué dans LHD en conditions non-dénaturantes.

L'HDV se transmet surtout par le biais des aiguilles contaminées et le sang, donc via les porteurs du HDV ou du HBV.

En Amérique du Nord et en Europe de l'Ouest, l'hépatite D se rencontre donc surtout chez les utilisateurs de drogues intraveineuses, les hémophiles et ceux qui ont reçu de multiples transfusions.

L'épidémiologie et les modes de contamination se superposent en partie. On estime globalement à 5 % la proportion de porteurs d'Ag-HBs infectés par HDV. Cependant, des disparités de prévalence géographique et épidémiologique sont constatées.

Une haute prévalence de cette maladie, chez les porteurs du virus de l'hépatite B, existe dans certaines régions du globe incluant le bassin amazonien de l'Amérique du Sud, l'Afrique centrale, le sud de l'Italie et dans les pays du Moyen-Orient.

Sur le pourtour méditerranéen, tout particulièrement en Italie du Sud, en Grèce et au Moyen-Orient où la fréquence du portage chronique de l'HBV est intermédiaire (1 % à 5 %), l'infection par l'HDV est élevée. Dans ces régions, la transmission intra-familiale a été suggérée, argumentée par des études phylogénétiques de virus infectant les membres d'une même famille (Niro et al., 1999). En Italie du Sud, la prévalence chez le sujet Ag-HBs positif diminue, passant de 23 % en 1987 à 8 % en 2000 (Gaeta et al., 2000).

En Afrique et en Asie, où le portage chronique de l'HBV est de fréquence élevée (10 % à 20 %), ainsi qu'en Amérique du Sud et dans les îles du Pacifique, où elle est intermédiaire (1 % à 5 %), la répartition de l'HDV est paradoxalement disparate. En Afrique, les études de séroprévalence montrent une distribution très hétérogène des patients ayant des anticorps anti-HD, alors que la prévalence globale d'infection HBV, estimée par la détection de l'Ag-HBs, se stabilise entre 12 et 14 % (Roingeard et al., 1992). Ainsi, des taux variables de 4 % (zone Nord du Sénégal) à 44 % (banlieue de Dakar) font émerger de probables facteurs socioéconomiques impliqués dans la transmission.

Les études de prévalence de l'HDV sont à interpréter prudemment. En effet, dans les populations étudiées, il y a une inclusion préférentielle des patients atteints d'hépatopathies. Chez les patients atteints d'hépatite aiguë ou chronique, la prévalence de l'infection par HDV est plus importante que chez les porteurs chroniques asymptomatiques de l'HBV. De plus, l'enquête sérologique d'une infection HDV repose sur la détection de l'Ag-HD et des Ac anti-HD totaux dans le sérum. De ce fait, des infections aiguës bénignes, au cours desquelles une production transitoire isolée d'IgM anti-HD se développerait, ne seraient pas recensées.

L'HDV est responsable d'hépatites aiguës et chroniques. Ces infections sont particulièrement sévères et évoluent plus rapidement vers la cirrhose que les hépatites B seules. C'est l'une des raisons pour lesquelles le diagnostic fiable d'HDV associé à HBV est crucial.

L'infection par un HDV est dépendante de l'HBV. Les isolats HDV de régions géographiques différentes montrent une variabilité génétique. Actuellement trois génotypes sont identifiés et désignés génotype-I, -II et -III.

Le génotype sert à l'épidémiologie des transmissions virales, permet d'étudier la répartition géographique et pourrait être corrélé au pouvoir pathogène.

L'HDV ne se développe que chez les patients également infectés par l'HBV. Cette double infection découle soit d'une co-infection ou d'une surinfection :
- la co-infection est à l'origine d'une hépatite aiguë. Le diagnostic, évoqué au cours d'une cytolyse hépatique, repose sur la détection des marqueurs de l'HDV associée à la présence d'IgM anti-HBc. L'Ag-HBs, généralement présent serait exceptionnellement négatif, justifiant de répéter les prélèvements pour suivre la cinétique d'évolution des marqueurs. Il est classique de constater une inhibition de réplication de l'HBV par l'HDV. Les IgM anti-HBc témoignent de l'infection récente par l'HBV. L'Ag-HD, très précoce, est rarement détecté, compte tenu de sa fugacité. Les anticorps apparaissent 2 à 3 semaines après le début des symptômes : les IgM anti-HD prédominent, mais gardent un titre modéré (<1:1000). Deux pics d'élévation des transaminases, séparés de deux à cinq semaines s'observent dans 10 à 20 % des co-infections, reflétant probablement différentes cinétiques de réplication des virus. La co-infection est donc caractérisée par une sévérité de l'hépatite aiguë souvent supérieure à celle occasionnée par l'HBV isolé. Ainsi, des hépatites fulminantes sont décrites en Amérique du sud et en Afrique Sub-saharienne ou dans certaines populations. L'évolution est généralement marquée par une résolution de l'hépatite après la phase aiguë et à l'image de l'histoire naturelle de l'HBV, seuls 5 % des patients co-infectés évoluent vers la chronicité.
- La surinfection est caractérisée par l'apparition d'une séroconversion HDV chez un patient porteur chronique de l'Ag-HBs. La virémie HDV précède l'apparition d'anticorps anti-HDV en l'absence de détection des IgM anti-HBc. La détection de ces marqueurs peut précéder une élévation des transaminases de plusieurs mois. En phase aiguë, la surinfection se traduit par une hépatite fulminante dans plus de 10 % des cas. De plus, passée la phase aiguë, la surinfection entraîne fréquemment (60 à 70 %) une hépatite chronique active avec évolution rapide vers la cirrhose. A la phase aiguë de la surinfection, la détection de l'Ag-HD est rapidement suivie de l'apparition des anticorps qui persistent à des taux élevés. Contrairement aux modèles classiques d'infection virale, IgG anti-HD et IgM anti-HD sont simultanément détectés dans les hépatites B-delta chroniques.

Le Tableau II ci-après résume l'évolution des marqueurs B et delta au cours des co-infections et des surinfections

| Co-infectionpar l'HDV | Phase aiguë | Évolution | |
|---|---|---|---|
| | | Chronique | Guérison |
| Ag-HBs | + | + | - |
| IgM anti-HBc | + | - | - |
| Ag-HD | +/- | - | - |
| IgM anti-HD | +/- | + | - |
| IgG anti-HD | +/- | + | + |
| ARN HDV | + | + | - |
| Ag-HD intrahépatique | + | + | - |

| Surinfection par l'HDV | | Évolution | |
|---|---|---|---|
| | | Chronique | Guérison |
| Ag-HBs | + | + | - |
| IgM anti-HB c | - | - | - |
| Ag-HD | +/- | - | - |
| IgM anti-HD | + | + | - |
| IgG anti-HD | + | + | + |
| ARN HDV | + | + | - |
| Ag-HD intra-hépatique | + | + | - |

Co-infection et surinfection sont cliniquement indistinctes. Le diagnostic virologique repose habituellement sur les différents marqueurs sériques. Plus rarement, l'Ag-HD peut être détecté sur les coupes anatomopathologique de la biopsie de foie.

Les marqueurs permettent de suivre l'évolution de la maladie vers la guérison ou la chronicité, de décider la mise sous traitement d'un malade et d'en évaluer l'efficacité.

L'HDV ne s'isole pas en culture cellulaire et le diagnostic repose donc essentiellement sur la recherche de l'Ag-HD (ELISA, IF) ou du génome viral (hybridation, PCR, PCR temps réel) pour les techniques directes et sur la détection des anticorps IgM anti-HD et IgG anti-HD pour les méthodes indirectes (ELISA).
- La recherche de l'Ag-HD intrahépatique peut être discutée dans les hépatites fulminantes compte tenu de la cinétique d'apparition des marqueurs sériques. Cet examen a valeur de référence pour étudier la réplication de l'HDV, mais ne peut pas être utilisée en routine.
- L'Ag-HD sérique est recherché dans le sérum en présence d'un agent dissociant qui expose l'Ag-HD, inclus dans l'enveloppe virale portant l'Ag-HBs. La présence d'anticorps (Ac) anti-HD à taux élevé (hépatites chroniques) fixant les antigènes sériques gêne la détection. Les techniques de *Western blot* sont développées à visée de recherche. La présence du virus dans le sang est transitoire et limitée à la phase précoce de l'infection et la possibilité de détecter l'Ag-HD diminue dans les jours suivant l'apparition des symptômes.
- L'immunocapture est utilisée pour détecter les IgM anti-HD et la compétition pour les IgG anti-HD. Les techniques ELISA ont d'abord utilisé comme antigène l'Ag-HD de sérum ou de foie de patients ou d'animaux infectés. Les nouveaux tests reposent sur des Ag-HD recombinants ou des peptides de synthèse.
- Les techniques d'hybridation ou de RT-PCR permettent la détection de l'ARN génomique après extraction des acides nucléiques et dénaturation des structures secondaires. Plusieurs systèmes d'amorces ont été décrits : leur choix est déterminant car la variabilité génétique dans des régions dites conservées peut conduire à des faux négatifs si les amorces choisies ne sont pas appropriées aux souches virales circulantes. Le choix des amorces de PCR doit tenir compte de l'épidémiologie locale des génotypes décrits et il est primordial de bien connaître la répartition de ces génotypes dans le monde.

Toutefois, aussi bien en cas de co-infection qu'en cas de surinfection, l'Ag-HD est en fait difficile à détecter, alors que la virémie précède l'apparition des anticorps.

Dans ce contexte, et notamment en raison de la mise en évidence de nouveaux génotypes, des réactifs nucléiques et protéiques permettant le diagnostic du HDV, quel que soit le génotype, sont nécessaires.

En effet l'étude des séquences nucléotidiques de l'HDV par différentes équipes dans le monde n'a permis de différencier, jusqu'à présent, que trois génotypes distincts :
- le génotype-1, qui est le plus fréquent et le plus largement répandu dans le monde. Depuis sa description initiale (chimpanzé infecté expérimentalement) par Wang (Wang et al., 1986 ; Wang et al., 1987), plusieurs groupes ont séquencé le génome de l'HDV d'isolats géographiques différents. La première séquence d'un HDV chez l'homme a été décrite en 1987, aux Etats-Unis par S. Makino et al., chez un patient toxicomane (Makino et al., 1987). Le génotype-I est très largement répandu en Italie, aux Etats-Unis, Taiwan, Nauru, France, Liban, Chine (Makino et al., 1987 ; Chao et al., 1991 b ; Imazeki et al., 1991 ; Lee et al., 1992 ; Niro et al., 1997 ; et Shakil et al., 1997). A l'intérieur du génotype-I un pourcentage de similarité nucléotidique de plus de 85 % est décrit.
- Un isolat japonais (Imazeki et al., 1990 ; Imazeki et al., 1991) est le prototype d'un 2^{ème} sous-groupe d'HDV. Ce génotype-II qui n'avait été décrit dans un premier temps qu'au Japon et à Taiwan (Imazeki et al., précités ; Lee et al., 1996b) semble avoir une répartition géographique beaucoup plus large. En particulier, des séquences de génotype-I et de génotype-II provenant de Yakoutie (Russie) ont également été caractérisées. Enfin, certains auteurs s'appuient sur une diversité intra-génotypique permettant la division en sous-types IIA (Imazeki et al., 1990 ; Imazeki et al., 1991 ; Lee et al., 1996b), IIB (Wu et al., 1998 ; Sakugawa et al., 1999) et IIC. Dans certains pays, l'infection par des virus de génotype-II s'associerait à des hépatites moins sévères que celles causées par les HDV de génotype-I ou -III (Wu et al., 1995b).
- En 1993 un 3^{ème} groupe a été décrit pour des génomes de virus péruviens et colombiens (Casey et al., 1993a). Le génotype-III n'a été décrit qu'en Amérique du Sud, et plus particulièrement dans le Bassin Amazonien associé à des hépatites sévères, voire à des épidémies d'hépatites fulminantes avec stéatose microvésiculaire (Casey et al., 1993a ; Casey et al., 1996b) et à une morbidité et à une mortalité élevées. Dans cette région géographique, on observe que l'HDV de génotype-III est associé de manière privilégiée avec l'HBV de génotype F. D'autres isolats de ce groupe ont récemment été isolés au Vénézuela (Nakano et al., 2001).

En comparant l'ensemble des génomes, deux à quatre régions conservées sont décrites (Chao et al., 1991b). Deux sont constamment retrouvées et sont centrées autour des sites d'autoclivage des génomes et antigénomes impliqués dans les activités auto-catalytiques. Les deux autres régions conservées sont situées dans le cadre de lecture codant pour la protéine HD (Chao et al., 1991b).

Toutefois, les techniques de détection sont tributaires de la variabilité génétique du virus recherché ; les réactifs connus, notamment à partir des séquences spécifiques de génotype-I, -II ou -III, ne permettent pas de détecter les infections à HDV variant et notamment les HDV de génotype différent de ceux précités.

En conséquence, les techniques de détection précisées ci-dessus risquent d'aboutir à des résultats négatifs tant au niveau nucléique qu'en ce qui concerne la réponse en anticorps.

La mise en évidence et la prise en compte de nouveaux variants sont importantes pour mettre au point des réactifs de détection et de diagnostic des hépatites D (sérodiagnostic, PCR, hybridation), suffisamment sensibles et spécifiques, c'est-à-dire ne conduisant pas à des résultats faussement négatifs ou faussement positifs : en effet, une dissociation IgM anti-HD positif / ARN-HDV négatif peut, à l'heure actuelle, être observée dans le contexte d'une hépatopathie sévère.

Dans le cadre de leurs travaux, les Inventeurs ont maintenant caractérisé, de manière surprenante, que la diversité génétique de l'HDV était significativement plus importante qu'antérieurement décrit, ce qui a des conséquences sur la fiabilité du diagnostic.

Ils ont en particulier mis en évidence neuf nouvelles séquences complètes d'HDV (trois originaires de Yakoutie et six originaires d'Afrique), circulant également en Île de France et qui n'appartiennent à aucun des génotypes connus.

L'analyse de ces nouveaux isolats :
- confirme l'existence d'une variabilité beaucoup plus importante de l'HDV que celle qui était décrite jusqu'à présent,
- remet en question la classification des HDV en seulement trois génotypes
- a conduit les Inventeurs à proposer un algorithme de PCR-RFLP, à partir d'une région partielle du génome pour le génotypage HDV et
- a conduit les Inventeurs à mettre au point des réactifs adaptés à un diagnostic fiable des infections à HDV, et ce quel que soit le génotype, alors que précédemment, on observait de nombreux résultats faussement négatifs (existence de nouveaux génotypes).

Les Inventeurs se sont donc donné pour but de pourvoir à des molécules d'acides nucléiques d'HDV, aptes à permettre la détection d'un HDV variant par rapport aux trois génotypes antérieurement décrits.

La présente invention a donc pour objet des molécules d'acide nucléique isolées, caractérisées en ce qu'elles sont sélectionnées dans le groupe constitué par :
- le génome complet du variant d'HDV dénommé dFr48 qui présente la séquence SEQ ID NO: 21 et
- le génome d'un HDV qui présente une divergence ou distance génétique ≤ 15 % avec la séquence SEQ ID NO: 21.

Selon un mode de réalisation avantageux desdites molécules, la région R0 est de préférence obtenue par amplification de l'ARN HDV avec les amorces 900S (SEQ ID NO:33) et 1280AS (SEQ ID NO:34).

Au sens de la présente invention, on entend par molécule d'acide nucléique, une molécule d'ADNc ou d'ARN présentant l'une des séquences génomiques d'HDV telles que définies ci-dessus et leurs séquences complémentaires sens et anti-sens.

La présente invention a également pour objet des molécules d'acide nucléique isolées qui comprennent au moins l'un des fragments des séquences d'un HDV variant telles que définies ci-dessus, sélectionnées dans le groupe constitué par :
a) le fragment R0 du HDV variant isolé suivant : dFr48, qui présente la séquence suivante : SEQ ID NO:50,
b) le fragment R1 qui s'étend des positions 307 à 1289 du génome d'HDV,
c) le fragment R2 qui s'étend des positions 889 à 328 du génome d'HDV,
d) le fragment R3 qui s'étend des positions 1486 à 452 du génome d'HDV,
e) le fragment R'1 qui s'étend des positions 305 à 1161 du génome d'HDV,
f) le fragment R'2 qui s'étend des positions 984 à 331 du génome d'HDV,
g) le fragment R644 qui s'étend des positions 889 à 446 du génome d'HDV,
h) le fragment G910 qui s'étend des positions 1206 à 929 du génome d'HDV,
i) le fragment p910 qui s'étend des positions 553 à 1550 du génome d'HDV,
j) l'ADNc codant pour la protéine sHD, de séquence SEQ ID NO:24,
k) l'ADNc codant pour la protéine LHD, de séquences SEQ ID NO:22 et

Au sens de la présente invention, les positions des fragments dans le génome d'HDV sont indiquées sur le génome circulaire en orientation génomique, selon la numérotation de Wang et al., 1986 ou 1987.

L'invention englobe également des fragments nucléotidiques complémentaires des précédents ainsi que des fragments modifiés, par rapport aux précédents, par enlèvement, ou addition de nucléotides dans une proportion d'environ 15 %, par rapport à la longueur des fragments ci-dessus et/ou modifiés au niveau de la nature des nucléotides dès lors que les fragments nucléotidiques modifiés conservent une capacité d'hybridation avec les séquences d'ARN génomiques ou antigénomiques des isolats tels que définis ci-dessus.

En effet, ces différentes souches virales, chez un même patient, à un moment donné, montre une population hétérogène de molécules d'ARN HDV ; en outre, au cours d'une infection chronique, en plus des hétérogénéités observées au niveau du site d'édition (position 1012), des mutations sont susceptibles d'apparaître. Les séquences virales semblent évoluer au sein de populations virales avec un taux de substitution variable de 3.10-2 à 3.10⁻³ par nucléotide et par an.

Certains de ces fragments sont spécifiques et sont utilisés comme sonde ou comme amorces ; ils s'hybrident spécifiquement à une souche d'HDV variante telle que définie ci-dessus ou à une souche apparentée ; on entend par HDV apparenté à un variant tel que défini ci-dessus, un HDV présentant une divergence génétique ≤ à 15 %.

De tels fragments sont utilisés pour la détection et le suivi épidémiologique des infections à HDV. Par exemple, le fragment R0 est utilisé pour la détection (RT-PCR) et le génotypage (PCR-RFLP) d'HDV. Les autres fragments qui couvrent la totalité du génome d'HDV sont utilisés pour la caractérisation moléculaire des HDV variants ; l'analyse phylogénétique de la séquence complète du génome ou de fragments de celui-ci correspondant en particulier à R0 ou à R2 permettent de rattacher les profils observés en PCR-RFLP à un génotype donné ou de caractériser de nouveaux génotypes.

En conséquence, la présente invention a également pour objet une méthode de détection d'un HDV variant selon l'invention, par hybridation et/ou amplification, réalisée à partir d'un échantillon biologique, laquelle méthode est caractérisée en ce qu'elle comprend :
(1) une étape d'extraction de l'acide nucléique à détecter, appartenant au génome du virus éventuellement présent dans l'échantillon biologique,
(2) la réalisation d'au moins une amplification génique à l'aide d'une paire d'amorces sélectionnée dans le groupe constitué par les amorces aptes à amplifier l'une des régions suivantes de l'ARN génomique d'HDV : R0, R1, R2, R3, R644, G910, p910, R'1 et R'2 et
(3) l'analyse du produit amplifié par comparaison avec l'une des molécules de séquences SEQ ID NO:21 correspondant au génome complet du variant dénommé, dFr48.

De manière avantageuse, l'étape (3) d'analyse peut être mise en oeuvre par restriction, séquençage ou hybridation ; dans ce dernier cas, la sonde utilisée (notamment dans des puces à ADN) sera avantageusement un fragment de 15 à 20 nucléotides, spécifique desdits fragments amplifiés.

Selon un mode de mise en oeuvre avantageux de ladite méthode, les amorces spécifiques pour l'amplification des régions R0, R1, R2, R3, R644, G910, p910, R'1 et R'2, mises en oeuvre à l'étape (2) sont sélectionnées dans le groupe constitué par :
- les amorces 900S (SEQ ID NO:33) et 1280AS (SEQ ID NO:34), pour l'amplification de R0 (environ 400 pb),
- les amorces 320S (SEQ ID NO:39) et 1280AS (SEQ ID NO:34), pour l'amplification du fragment R1 (environ 960 pb),
- les amorces 900S (SEQ ID NO:33) et 320AS (SEQ ID NO:45), pour l'amplification de R2 (environ 1100 pb), qui contient le gène sHD correspondant aux positions 1598-950,
- les amorces 1480S (SEQ ID NO:46 ) et 440AS (SEQ ID NO:47),, pour l'amplification de R3 (environ 650 pb),
- les amorces 900S (SEQ ID NO:33) et 420AS (SEQ ID NO:40), pour l'amplification de la région R644 (environ 1250 pb) de l'isolat dFr644,
- les amorces 318S (SEQ ID NO:35) et 1150AS (SEQ ID NO:36), pour l'amplification de R'1 (environ 850 pb),
- les amorces 960S (SEQ ID NO:37) et 345AS (SEQ ID NO:38), pour l'amplification de R'2 (environ 1050 pb),
- les amorces R910S (SEQ ID NO:41) et R910As (SEQ ID NO:42 ) pour l'amplification de la région G910 (environ 1400 pb) de l'isolat dFr910,
- les amorces S1910R (SEQ ID NO:43) et As1910R (SEQ ID NO:44) pour l'amplification de la région p910 (environ 650 pb) de l'isolat dFr910.

La présente invention a également pour objet une méthode de détection et de génotypage d'HDV à partir d'un échantillon biologique, laquelle méthode est caractérisée en ce qu'elle comprend :
(a) une étape d'extraction de l'acide nucléique appartenant au génome du virus HDV,
(b) une étape d'amplification de la région R0 délimitée par les positions 889 à 1289 du génome HDV,
(c) un premier traitement des molécules d'acides nucléiques amplifiées par les enzymes de restriction *Sma*I et *Xho*I*,* pour produire un premier ensemble de fragments de restriction, et
(d) un deuxième traitement des molécules d'acides nucléiques par l'enzyme de restriction *Sac*II, pour produire un deuxième ensemble de fragments de restriction
(e) l'analyse combinée des deux ensembles de fragments de restriction produits par RFLP (*Restriction Fragment Length Polymorphism*), de manière à détecter la présence et/ou déterminer le type d'HDV présent dans ledit échantillon biologique.

Selon un mode de mise en oeuvre avantageux de ladite méthode, l'étape (b) d'amplification est avantageusement mise en oeuvre avec les amorces 900S (SEQ ID NO: 33) et 1280AS (SEQ ID NO:34).

La méthode selon l'invention permet de définir de nouveaux profils de restriction et de classer les HDV en sept génotypes distincts.

Selon un autre mode de mise en oeuvre avantageux de ladite méthode, elle comprend en outre :
(f) l'amplification des molécules d'acide nucléique dudit échantillon par RT-PCR avec les amorces 900S (SEQ ID NO:33) et 320AS (SEQ ID NO: 45) de manière à amplifier la région R2 et
(g) le séquençage direct de la région R2 amplifiée et la comparaison avec l'une des molécules d'ARN de séquences SEQ ID NO: 1, 6, 11, 16, 21 et 26, correspondant respectivement aux génomes complets des isolats ou variants dénommés respectivement dFr45, dFr47, dFr73, dFr910, dFr48 et dFr644, par exemple par analyse phylogénétique.

Lorsque des profils inhabituels sont observés, cette étape supplémentaire permet de caractériser de nouveaux génotypes. En effet, ces analyses complémentaires de la PCR-RFLP permettent de rattacher le nouveau profil observé à un génotype donné, ou de caractériser un nouveau génotype, par analyse phylogénétique.

La présente invention a également pour objet un vecteur recombinant, notamment un plasmide, comprenant un insert constitué par une molécule d'acide nucléique telle que définie ci-dessus.

La présente invention a également pour objet une cellule transformée par une molécule d'acide nucléique telle que définie ci-dessus.

La présente invention a églament pour objet des produits de traduction codés par l'une des molécules d'ARN de séquences SEQ ID NO: 21 correspondant à l'ARN génomique complet du variant dénommé dFr48 ou par sa séquence complémentaire sens ou anti-sens.

La présente invention a également pour objet les protéines codées par le génome d'un HDV variant tel que défini ci-dessus.

Selon un mode de réalisation avantageux de l'invention, ladite protéine est sélectionnée dans le groupe constitué par :
- la protéine LHD de dFr48 qui présente respectivement la séquence SEQ ID NO:23 et
- la proteine sHD de dFr48 qui présente la séquence SEQ ID NO:25.

La présente invention a également pour objet un peptide, caractérisé en ce qu'il est constitué par un fragment d'une protéine telle que définie ci-dessus, sélectionné dans le groupe constitué par:
- le peptide A constitué par les 19 acides aminés de l'extrémité carboxy-terminale des séquences SEQ ID NO:23,
- le peptide C constitué par les 9 acides aminés précédents la séquence SEQ ID NO:59 (SEQ ID NO:64).

De tels peptides sont utiles pour le diagnostic indirect (sérologie) d'une infection par HDV, notamment par une méthode immunoenzymatique (ELISA) :
- le peptide B, qui est conservé, permet la détection de l'ensemble des variants selon l'invention et du génotype II d'HDV, et
- le peptide C est spécifique des différents variants d'HDV selon l'invention.

La présente invention a en outre pour objet l'utilisation d'une molécule d'acide nucléique telle que définie ci-dessus ou d'une protéine telle que définie ci-dessus pour la préparation d'un kit de détection et de génotypage d'un HDV.

Outre les dispositions qui précèdent, l'invention comprend encore d'autres dispositions, qui ressortiront de la description qui va suivre, qui se réfère à des exemples de mise en oeuvre de la présente invention ainsi qu'aux dessins annexés dans lesquels :

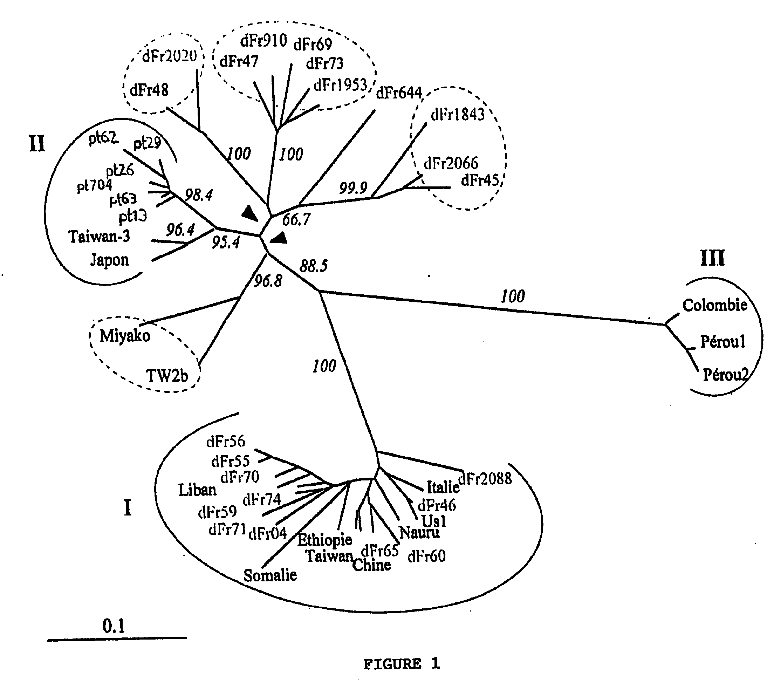

- la figure 1 représente l'arbre phylogénétique de la région R0 obtenu par la méthode du plus proche voisin *"neighbor-joining".* Les nombres en italique indiquent les valeurs de bootstraping (BV) sur 10⁴ rééchantillonnages et le signe ◢ indique les BV < 50 %. L'échelle représente le nombre de substitution de nucléotides par site.

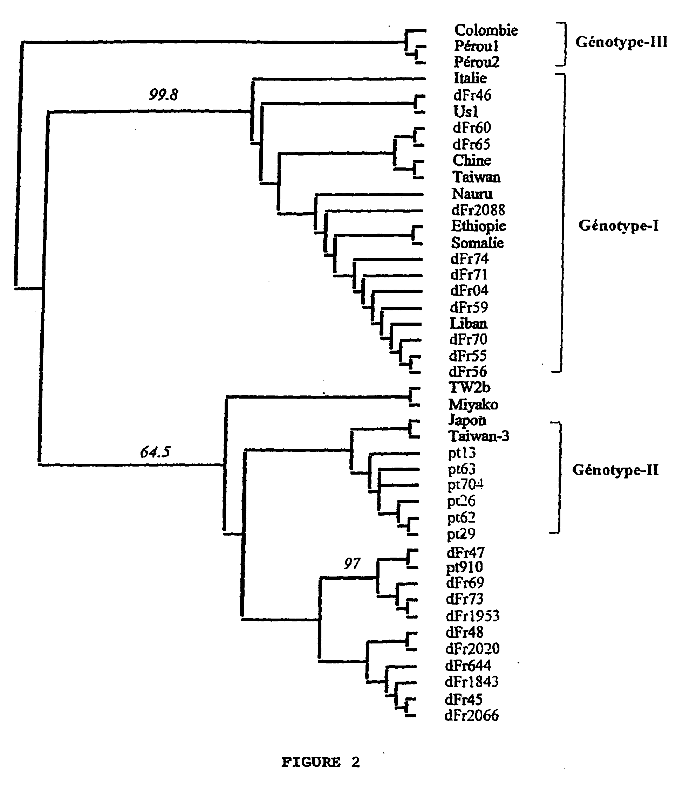

- la figure 2 représente l'arbre phylogénétique des régions R0 d'HDV obtenu par la méthode de maximum de parcimonie. Les isolats Pérou1, Pérou2 et Colombie sont choisis comme "outgroup". Les chiffres en italique indiquent les valeurs de bootstraping (BV) sur 10⁴ rééchantillonnages,

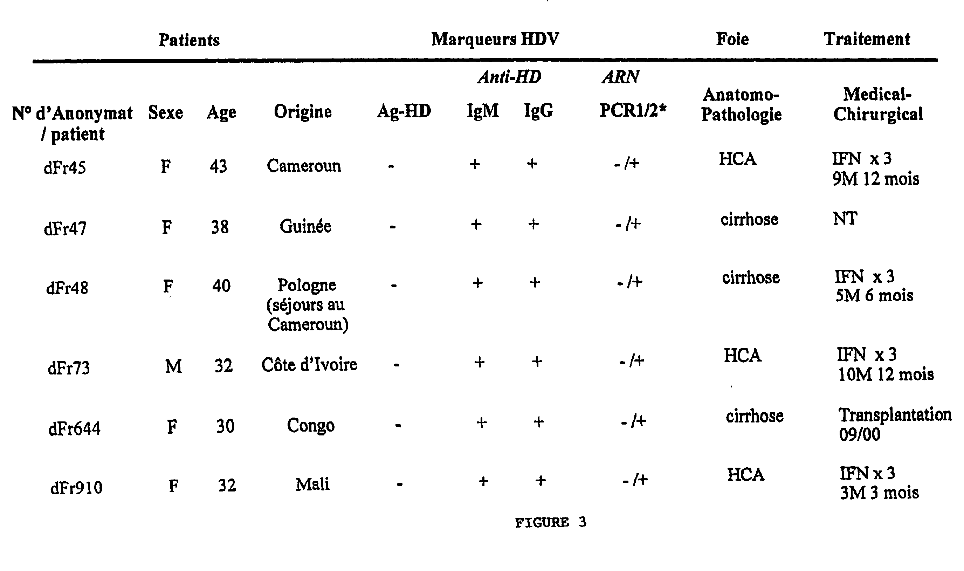

- la figure 3 illustre les données cliniques de chacun des six patients infectés par les isolats d'HDV d'origine africaine. * indique respectivement les PCR 6S/6As et PCR R0,

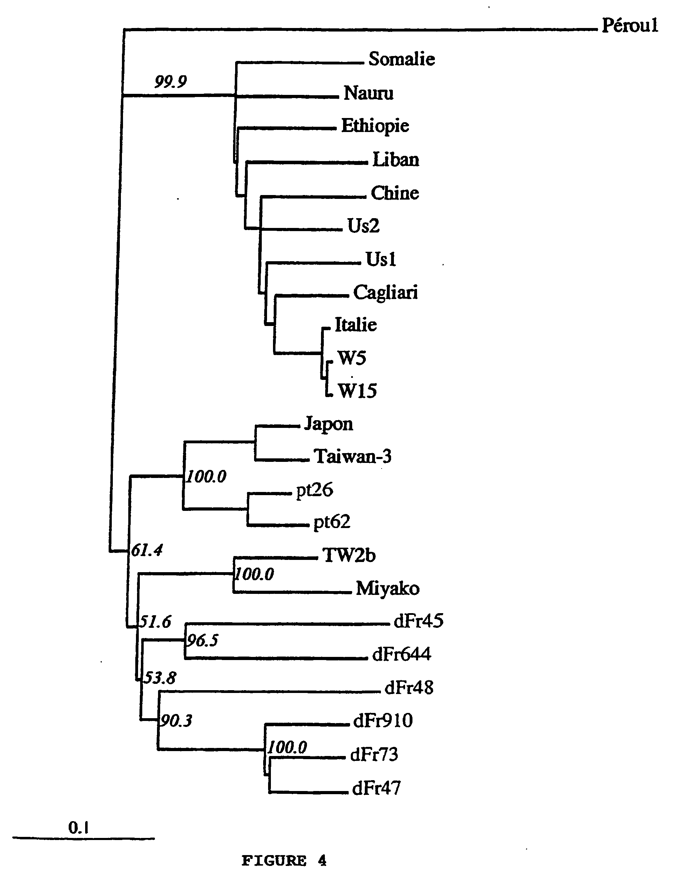

- la figure 4 représente l'arbre phylogénétique des génomes complets d'HDV, obtenu par la méthode du plus proche voisin "*neighbor-joining*"*.* Les nombres en italique, au niveau de chaque noeud, indiquent les valeurs de bootstraping (BV) sur 10⁴ rééchantillonnages. L'échelle représente le nombre de substitution de nucléotides par site.

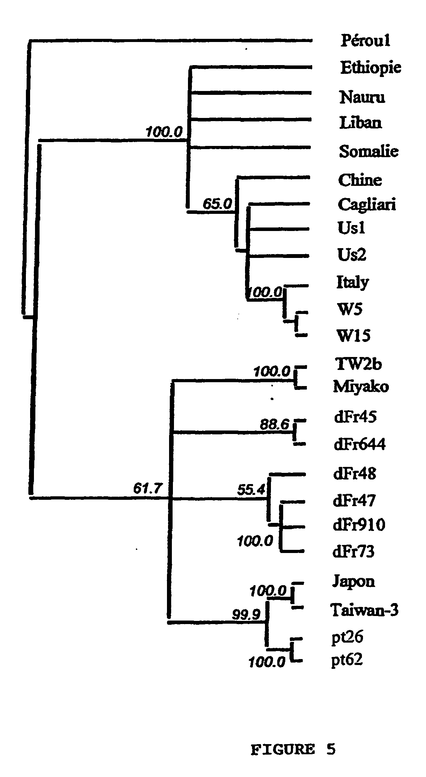

- la figure 5 représente l'arbre phylogénétique des génomes complets d'HDV, obtenu par la méthode de maximum de parcimonie. Les nombres en italique, au niveau de chaque noeud, indiquent les valeurs de bootstraping (BV) sur 10⁴ rééchantillonnages,

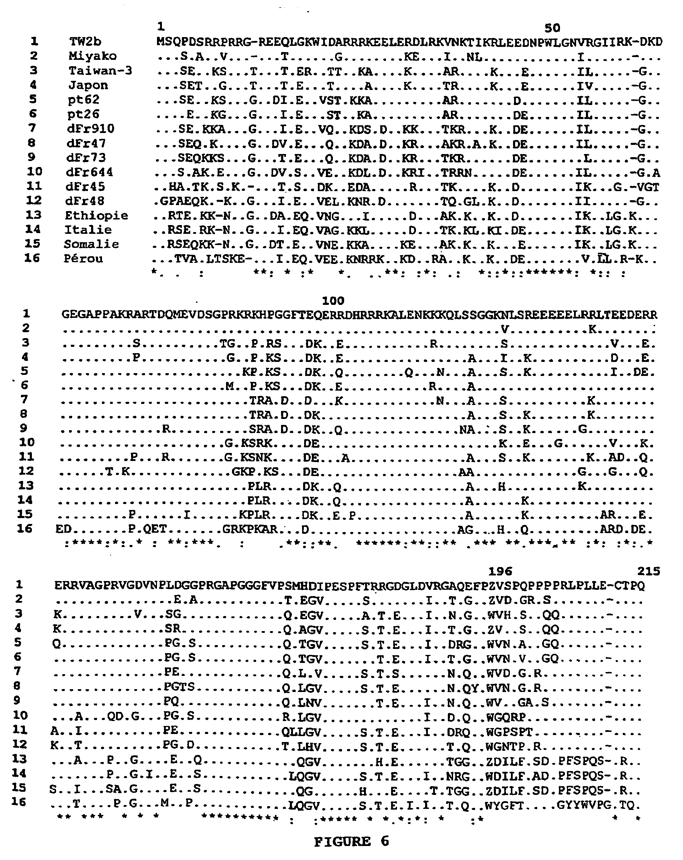

- la figure 6 représente l'alignement des séquences en acides aminés des protéines delta des six isolats d'origine africaine (lignes 7, 8, 9, 10, 11 et 12) avec les séquences connues de génotype I (lignes 13, 14 et 15), génotype II (lignes 3, 4, 5 et 6), génotype III (ligne 16) et TW2b/Miyako (lignes 1 et 2), à l'aide du logiciel Clustal (version 1.8). L'acide aminé en position 196 de la protéine p27 correspond au codon de terminaison de la protéine p24 (Z) ou au codon tryptophane (W) qui conduit à la synthèse de la protéine p27 qui s'étend des acides aminés 1 à 215.

Il doit être bien entendu, toutefois, que ces exemples sont donnés uniquement à titre d'illustration de l'objet de l'invention, dont ils ne constituent en aucune manière une limitation.

### EXEMPLE 1 : MATERIELS ET METHODES

### 1- Patients et échantillons.

22 sérums provenant de sujets suivis dans les différents centres hospitaliers de la région parisienne ont été analysés. Les patients étaient porteurs chroniques de l'Ag-HBs. Le diagnostic de l'infection delta a été effectué par la recherche des marqueurs sérologiques (Ag-HD, anti-AgHD de type IgM et IgG) et la détection du génome viral de l'HDV par RT-PCR. L'Ag-HD n'a été détecté dans aucun des sérums analysés. Les anticorps anti-AgHD de type IgM témoignant de la chronicité de l'infection delta, et de type IgG ont été retrouvés chez tous les patients. La totalité du génome de HDV a été caractérisée sur six patients. Tous les sérums étaient conservés à -80°C jusqu'à l'extraction de l'ARN viral.

### 2- Extraction de l'ARN HDV.

Pour extraire l'ARN HDV, un volume de 250 µl de sérum est ajouté à 75 µl de TRIzol LS Reagent (Gibco BRL, Life Technologies). Après 30 secondes d'homogénéisation, le mélange est incubé pendant 5 min à température ambiante. L'extraction des lipides s'effectue en ajoutant 200 µl de chloroforme refroidi à +4°C. Après une nouvelle homogénéisation au vortex, les tubes sont incubés puis centrifugés à 14 000 rotations par minute (rpm) pendant 10 min, à +4°C. La phase aqueuse est transférée dans des tubes d'extraction et les ARNs sont précipités par 500 µl d'isopropanol froid, en présence d'1 µg de glycogène. Après 15 min d'homogénéisation, les échantillons sont centrifugés à 14 000 rpm pendant 10 min, à +4°C. Après rinçage à l'éthanol à 70 %, les tubes sont à nouveau centrifugés à 14 000 rpm pendant 10 min, à + 4°C. Les culots sont séchés sous une hotte à température ambiante, puis repris dans 100 µl d'eau stérile comprenant un inhibiteur de ribonucléases (RNasin, Promega). A cette étape, des précautions sont prises pour éviter d'éventuelles contaminations des tampons et des échantillons par les ribonucléases.

### 3- Synthèse d'un ADN complémentaire (ADNc).

Cette étape consiste à synthétiser un brin d'ADN complémentaire à l'ARN HDV par transcription inverse.

Pour éliminer la structure secondaire de l'ARN HDV, 5 µl d'ARN extrait précédemment sont ajoutés à un mélange réactionnel contenant 5 µl (ou 0,5 pmoles) de désoxynucléotides triphosphates (dNTP) et 1 µl (0,4 pmoles) d'hexanucléotides aléatoires. Les ARNs sont ensuite dénaturés pendant 3 min, à 95°C. Pour fixer les ARNs dénaturés, les tubes sont congelés immédiatement dans l'éthanol refroidi à -20°C. Dix µl d'un mélange réactionnel, contenant 2,5 µl de dithiothréitol (DTT), 100 unités (U) de transcriptase réverse Superscript II, (Gibco BRL, Life Technologies) et son tampon réactionnel, ainsi que 20 U d'inhibiteur de ribonucléases (RNasin, Promega) sont ajoutés à l'ARN dénaturé. La réaction de transcription inverse est effectuée à 42°C pendant 45 min, puis arrêtée par incubation à 94°C pendant 5 min. Les ADNc sont ensuite conservés à -80°C.

### 4- Amplification génique.

L'amplification des ADNc est effectuée, de manière exponentielle, par PCR (*Polymerase Chain Reaction*). Deux types de polymérases ont été utilisés : la polymérase AmpliTaq Gold (*Thermophilus aquaticus*) (PE Applied Biosystems) et la polymérase Pwo (*Pyrococcus woesi*) ou Expand^{™} High Fidelity PCR System (Roche).

L'amplification a été effectuée à partir de 5 µl d'ADNc qui sont ajoutés à un mélange réactionnel de PCR contenant : 0,25 pmoles/µl d'amorce sens et antisens (Tableau III), 200 µmoles de chaque dNTP, 1,5 mM de MgCl₂, 1 U d'AmpliTaq Gold ou 2,6 U de polymérase Expand^{™} en présence des tampons PCR correspondants. La réaction de PCR a été réalisée dans un thermocycleur (PCR Sprint, Hybaid, Coger), selon le protocole suivant : dénaturation des hybrides ADNc - ARN à 94°C pendant 9 min, suivie de 40 cycles successifs, chacun comprenant une dénaturation des ADNs à 94°C pendant 45 sec, une hybridation des amorces (900S/1280As ou 6S/6As) à 58°C pendant 30 sec, synthèse du brin complémentaire grâce à la polymérase par élongation à 72°C pendant 45 sec. Enfin, une élongation finale à 72°C pendant 4 min 30 sec à 72°C.

**Tableau III : Séquences des amorces utilisées pour les réactions de PCR et leur position sur le génome HDV.**

| **Amorces** | **Séquence 5' -> 3'** | **numéro d'identification** | **positions*** |
|---|---|---|---|
| **6S** | gaggaaagaaggacgcgagacgcaa | SEQ ID NO:31 | 904-929 |
| **6As** | accccctcgaaggtggatcga | SEQ ID NO: 32 | 1141-1121 |
| **900S** | catgccgacccgaagaggaaag | SEQ ID NO: 33 | 889-911 |
| **1280As** | gaaggaaggccctcgagaacaaga | SEQ ID NO:34 | 1289-1265 |
| **318S** | ctccagaggaccccttcagcgaac | SEQ ID NO:35 | 305-328 |
| **1150As** | cccgcgggttggggatgtgaaccc | SEQ ID NO:36 | 1161-1138 |
| **960S** | gtacactcgaggagtggaaggcg | SEQ ID NO:37 | 962-984 |
| **345As** | tctgttcgctgaaggggtcct | SEQ ID NO:38 | 331-311 |
| **320S** | ccagaggaccccttcagcgaac | SEQ ID NO 39 | 307-328 |
| **420As** | aacaccctcctgctagcccc | SEQ ID NO: 40 | 446-427 |
| **R910S** | ccggagttcctcttcctcctcc | SEQ ID NO:41 | 1206-1227 |
| **R910AS** | gttcgcgtcegagtccttettte | SEQ ID NO:42 | 929-907 |
| **S1910R** | gagctttcttcgattcggac | SEQ ID NO:43 | 1531-1550 |
| **AS1910R** | gactggtcccctcatgttcc | SEQ ID NO:44 | 572-553 |

| | | | |
|---|---|---|---|
| * Selon la numérotation de Wang et al. (Nature, 1986, 323, 508-514 ; Nature, 1987, 328, 456) | | | |

### 4.1 - Stratégie d'amplification du génome viral HDV

Le couple d'amorces 6S et 6As permet d'amplifier un fragment d'ADN correspondant à l'extrémité carboxy-terminale du gène codant pour l'antigène delta.

La région R0 comprenant l'extrémité carboxy-terminale du gène codant pour l'Ag-HD et une partie de la région non codante a été amplifiée pour tous les échantillons à l'aide des amorces 900S (SEQ ID NO:33) et 1280As (SEQ ID NO:34). L'amorce 900S utilisée a été délétée de 7 nucléotides à l'extrémité 5', par rapport à celle utilisée par Casey et al., 1993a, précité, pour la classification des génotypes d'HDV.

La sélection de ces amorces permet, de manière surprenante, l'amplification d'un fragment apte à permettre de distinguer les génotypes connus (I, II et III) de nouveaux génotypes.

Les séquences complètes du génome viral HDV de quatre échantillons (*dFr45, dFr47, dFr48 et dFr73*) ont été obtenues par amplification de deux régions chevauchantes R'1 (850 bases) et R'2 (1050 bases), respectivement, à l'aide des couples d'amorces 318S/1150AS et 960S/345AS.

Pour l'échantillon *dFr644,* la variabilité observée dans la région correspondant aux amorces précédemment décrites a conduit à amplifier la région 644 (R644) à l'aide d'un couple d'amorces spécifique : 900S et 480AS.

Pour l'échantillon *dFr910,* la séquence nucléotidique R0, a permis de définir des amorces nouvelles et spécifiques de l'échantillon pour amplifier le génome complet. Deux couples d'amorces ont été choisis : les amorces R910S et R910AS, amplifiant un fragment de 1400 bases correspondant à la région G910. Un autre couple d'amorces S 1910R et AS 1910R amplifiant un fragment de 650 bases (région p910) a été indispensable pour couvrir la totalité du génome.

L'amplification des différentes régions (R1, R2, R3, R644, R'1, R'2, G910 et p910) est effectuée comme décrite précédemment. Les températures d'hybridation et d'élongation ainsi que le temps d'élongation utilisés pour chacune des PCR, sont indiqués dans le Tableau IV.

**Tableau IV :Amplification des différents fragments du génome**

| Régions amplifiées | Taille des fragments (bases) | Température d'hybridation (°C) | Température d'élongation (°C) | Temps d'élongation |
|---|---|---|---|---|
| RI | 960 | 62 | 72 | 1 min 15 s |
| R2 | 1100 | 56 | 72 | 1 min 30 s |
| R3 | 650 | 50 | 72 | 1 min |
| R644 | 1250 | 58 | 72 | 40 s |
| G910 | 1400 | 58 | 72 | 1 min 40 s |
| p910 | 650 | 58 | 72 | 1 min |
| R'1 | 850 | 63 | 72 | 1 min |
| R'2 | 1050 | 60 | 72 | 1 min 20 s |

### 5-Analyse des produits d'amplification.

Un volume de 8 µl du produit PCR est déposé en présence de 2 µl de solution de dépôt dans un gel à 1,3 % d'agarose préparé dans du tampon Tris-Borate/EDTA 0,5X et contenant 0,5 µg/ml de bromure d'éthidium (BET). L'électrophorèse est effectuée en tampon TBE 0,5X. La migration est effectuée, en présence d'un marqueur de taille (Raoul™, Appligène). Le fragment amplifié est visualisée sous rayons ultraviolets à 312 nm et photographié.

### 6- Clonage et séquençage des génomes HDV.

Avant l'étape de clonage et de séquençage, les produits d'amplification ont été purifiés afin d'éliminer toutes traces de sels et d'enzymes.

### 6.1- Élongation par la Taq-polymérase standard.

Cette étape est effectuée dans le cas où l'amplification du produit a été réalisée avec la polymérase Pwo. Elle permet d'ajouter les résidus de désoxyadénosines (A) aux extrémités 3' des produits de PCR, du fait que la polymérase Pwo, dotée d'une activité exonucléasique 5'----> 3', diminue l'incorporation des désoxyadénosines.

Un volume de 10 µl d'ADN purifié est ajouté à un mélange réactionnel de 70 µl contenant : 0,2 mM de dNTP, 1,5 mM de MgCl₂, du tampon 1X et 2,5 U de Taq Polymérase (Perkin Elmer). L'élongation est effectuée à 72°C, pendant 30 minutes. Les produits de PCR subissent alors une nouvelle purification au phénol-chloroforme et une précipitation à l'éthanol, puis sont repris dans 10 µl d'eau stérile.

### 6.2- Clonage dans le vecteur pCRII-TA-cloning (Invitrogen).

Le clonage est utilisé pour confirmer la séquence nucléotidique de l'ADN amplifié. Il est réalisé en utilisant le vecteur pCRII (Invitrogen).

Le vecteur pCRII se présente sous forme linéaire. Il possède des résidus de désoxythymidine (T) permettant le clonage du produit amplifié grâce aux résidus complémentaires de désoxyadenosine (A) ajoutés par la Taq polymérase. Il possède également les séquences promoteurs Sp6 et T7, deux sites de restriction *Eco*RI qui encadrent le site d'insertion du produit de PCR et les gènes de résistance à l'ampicilline et à la kanamycine. Une fraction du gène *lac*Zα*,* codant pour la β-galactosidase, facilite la sélection des recombinants par la couleur des colonies. En effet, les plasmides ayant intégré l'insert n'expriment pas le gène *lac*Zα*.* Les colonies bactériennes sont alors blanches en présence de substrat de β-galactosidase (X-Gal ou 5-bromo 4-chloro 3-indoyl β-galactoside, Roche) et d'un inducteur du gène (IPTG ou isopropyl-thio β-D-galactoside, Roche). Ainsi, les bactéries recombinantes sont sélectionnées grâce à leur résistance à l'ampicilline et à un crible bleu-blanc.

Le rapport moléculaire choisi insert/vecteur est de 3/1 et le volume de produit PCR utilisé est variable, selon la quantité d'ADN estimée par électrophorèse en gel d'agarose comme décrit précédemment. Le mélange réactionnel de 10 µl contient 50 ng de vecteur pCRII, la quantité d'insert correspondante, 4 U de T4 DNA Ligase, et le tampon Ligase 1X. La réaction de ligature est conduite pendant 18 heures à 14°C. Les tubes sont ensuite conservés à +4°C.

Les bactéries *Escherichia coli* TOP10F' (Invitrogen), rendues compétentes par traitement au chlorure de calcium, sont conservées à -80°C, prêtes à l'utilisation. Un volume de 50 µl de bactéries compétentes est mis au contact de 3 µl de la solution de ligature pendant 30 minutes, dans de la glace. Un choc thermique (30 sec à 42°C) fait pénétrer l'ADN plasmidique dans les bactéries, qui sont remises immédiatement sur glace quelques minutes, avant d'être incubées 1 heure à 37°C, dans 250 µl de milieu SOC (Tryptone 2 % ; NaCl 10 mM ; KCl 2,5 mM ; MgCl₂ 10 mM; glucose 20 mM, extraits de levure 5 g/l). On isole ensuite les colonies sur boîtes de Pétri contenant du LB agar (milieu de Luria-Bertani), supplémenté en ampicilline (50 µl /ml), 40 µl de X-Gal (40 mg/ml) et 40 µl d'IPTG (100 mM) sont répartis.

### 6.3-Extraction plasmidique et analyse de l'insert.

Les colonies blanches sont ensemencées en bouillon LB-ampicilline (50 µl /ml) et incubées 18 heures, à 37°C, sous agitation. Une colonie bleue, c'est-à-dire n'ayant pas inséré de fragment est retenue comme témoin négatif de ligation.

L'extraction plasmidique est réalisée à l'aide d'un kit commercial QIAprep® Spin Miniprep (Qiagen). Brièvement, après centrifugation (3000 tours par min à +4°C) et élimination du surnageant, le culot bactérien est suspendu dans 250 µl de tampon (Tris-HCl 50 mM pH 8, EDTA 10 mM, RNase A 100 µl/ml) et lysé par ajout de 250 µl de tampon alcalin (NaOH 200 mM, SDS 1 %). Après 5 min d'homogénéisation, on ajoute 350 µl de tampon (acétate de potassium 3M, pH 5,5). Le surnageant contenant l'ADN plasmidique est ensuite transféré dans une colonne QIAprep. Une centrifugation élimine l'éluat dans le tube collecteur.

La colonne est lavée par un tampon éthanol, séchée, puis l'ADN est élué dans 50 µl d'eau stérile.

Pour vérifier l'insertion du fragment d'intérêt, le plasmide est alors digéré par l'enzyme de restriction EcoRI. La digestion est effectuée dans un mélange réactionnel de 30 µl contenant : 2 µl de la solution plasmidique, 10 U d'enzyme EcoRI

(Appligène) et du tampon réactionnel 1X. La digestion dure 2 heures à 37°C et le résultat est visualisé par électrophorèse en gel d'agarose.

### 6.4- Séquençage par la méthode BigDye Terminator

Le séquençage est réalisé sur les produits PCR préalablement purifiés sur colonnes Microcon 50 (Amicon) ou sur l'ADN plasmidique. Les fragments sont, soit séquencés directement avec les amorces de PCR (fragment R0 séquencé avec les amorces 900S et 1280As) soit après clonage dans le vecteur pCRII en utilisant les amorces universelles (Sp6 et T7).

Deux clones différents ont été retenus pour chacune des régions amplifiées, afin de lever d'éventuelles ambiguïtés lors de la lecture des séquences nucléotidiques.

Le séquençage est effectué à l'aide du réactif BigDye Terminator (PE, Applied Biosystems). Le principe de séquençage consiste en une électrophorèse verticale en gel de polyacrylamide de l'ADN marqué par quatre fluochromes différents. Les matrices d'ADN sont déposées sur le gel et séparées selon leur taille, avant de soumettre le gel à un faisceau laser en continu. Le laser excite les fluochromes qui émettent chacun à une longueur d'onde différente, détectée par un spectrographe. Un logiciel informatique, couplé au séquenceur, permet l'analyse automatique et la conversion des données en séquences nucléotidiques.

Le mélange réactionnel de 10 µl comprend : 4 µl de la solution de marquage (désoxynucléosides triphosphates (dATP, dCTP, dGTP, dUTP), AmpliTaq DNA polymérase, MgCl₂, Tampon Tris-HCl pH 9), 20 pmoles d'amorce (sens ou anti-sens) et 500 ng de plasmide purifié sur colonnes de type Centricon. Les réactions de séquences (sens et antisens) sont effectuées dans un thermocycleur de type Perkin 9600, avec 25 cycles (96°C pendant 10 sec ; 50°C pendant 5 sec ; 60°C pendant 4 min). Les produits sont ensuite précipités dans 40 µl d'éthanol à 70 %, déposés sur gel et analysés grâce à un séquenceur automatique de type ABI PRISM 377.

Les séquences brutes obtenues se présentent sous forme d'électro-phorégrammes. La validation et l'exploitation des séquences se fait à l'aide du Logiciel Séquence Navigator (PE, Applied Biosystems). Elles font l'objet d'au moins une double lecture, par deux amorces de séquençage différentes (Sens et Antisens), afin de minimiser les erreurs.

Ces séquences sont ensuite directement saisies sur un ordinateur en utilisant le logiciel *DNA Strider 1.3* permettant une analyse rapide des séquences.

### 7- Analyse informatique des séquences nucléotidiques et protéiques

Les séquences lues et corrigées sont comparées et soumises aux différents algorithmes phylogénétiques.

Les séquences obtenues (22 séquences) ont été comparées à 21 séquences génomiques complètes d'HDV disponibles dans GenBank (Tableau V).

**Tableau V : Numéros d'accession des différents isolats**

| **Numéros d'accès (GenBank)** | **Nom d'isolat** | **Origine géographique** |
|---|---|---|
| 1 X04451 | Italy 1 (A20) | Italie |
| 2 M84917 | Lebanon 1 | Liban |
| 3 X85253 | PatientA. | Cagliari (Italie) |
| 4 X60193 | 7/18/83 (patient S) | (patient S) Japon |
| 5 M92448 | Taiwan | Taiwan |
| 6 L22061 | Columbia | Colombie |
| 7 X77627 | Chinese human serum | Chine centrale |
| 8 L22064 | Peru-2 | Pérou |
| 9 L22063 | Peru-1 | Pérou |
| 10 L22066 | US-2 | États-Unis |
| 11 M58629 | Nauru | Île de Nauru |
| 12 U81988 | Somalia | Somalie |
| 13 U81989 | Ethiopia 1 | Ethiopie |
| 14 AF098261 | Canada | Canada(Quebec) |
| 15 U19598 | Taiwan 3 | Taiwan |
| 16 AF018077 | TW2b | Taiwan |
| 17 L22062 | Japan 3 | Japon |
| 18 AF309420 | Miyako | Ile de Miyako (Okinawa, Japon) |
| 19 D01075 | Us- 1 | États-Unis |
| 20 M21012 | W 15 | Transmission expérimentale (Marmotte) |
| 21 AJ307077 | W5 | Transmission expérimentale (Marmotte) |
| 22 AJ309868 à AJ309881 | Isolats de Yakoutie | Yakoutie (Russie) |
| | | |

La première étape consiste globalement à aligner les séquences d'intérêt avec les séquences HDV de référence décrites et répertoriées dans la banque de données (Genbank), en utilisant le programme CLUSTAL W1.8 (Thompson et al., N.A.R., 1994, 22, 4673-4680). Des corrections manuelles mineures ont parfois été nécessaires à l'aide du logiciel *SeqPup* afin d'optimiser l'alignement.

Deux approches ont été suivies : l'utilisation de l'alignement des protéines pour le gène HD et l'étude de la stabilité des positions alignées à l'aide d'un logiciel d'alignement adapté.

A partir de cet alignement de séquences nucléotidiques, des arbres phylogénétiques sont construits par différents algorithmes. Les analyses sont basées sur des matrices de distances (approche phénétique), des calculs de maximum de parcimonie (MP ; approche cladistique) et de maximum de vraisemblance (ML ; approche statistique).

### - approche phénétique (distance génétique)

Le principe de cette méthode est de trouver des paires de séquences voisines, en minimisant la longueur totale des branches de l'arbre. Cette approche permet de reconstruire une phylogénie sur la base du calcul de la ressemblance globale entre les séquences comparées deux à deux qui est exprimée par une distance. C'est une méthode qui permet de convertir les données de séquences en valeurs numériques de distances, arrangées en matrice. La topologie de l'arbre est construite de manière à regrouper les séquences qui possèdent le plus de caractères en commun, en utilisant une des méthodes de regroupement comme la méthode du plus proche voisin ou *"neighbor-joining"* (Saitou et al., 1987).

### - approche cladistique (maximum de parcimonie)

Le principe de cette méthode consiste à établir des relations de parenté entre les séquences par la recherche de bases nucléotidiques partagées, en minimisant les évènements génétiques. L'algorithme de maximum de parcimonie construit un arbre phylogénétique de telle manière qu'il implique le minimum de mutations. L'arbre retenu est celui qui nécessite le moins de changement. Cette méthode est sensible aux différences de taux de mutations le long des branches. Les "clades" ou "groupes monophylétiques" sont constitués par les groupes de séquences partageant un ancêtre commun, à l'exclusion de toute autre séquence.

### - approche statistique

La méthode de maximum de vraisemblance est considérée comme une approche statistique. Le programme calcule la probabilité pour qu'une séquence évolue vers une autre dans le temps. En d'autres termes, il consiste à considérer les changements au niveau de chaque site ou caractère comme des évènements probabilistes indépendants. Cet algorithme de vraisemblance est cumulé sur tous les sites, et la somme est maximisée pour estimer la longueur de branche de l'arbre. Cette méthode nécessite un temps de calcul important pour rechercher l'arbre phylogénétique, le plus vraisemblable, correspondant aux séquences observées du fait qu'elle prend en compte la probabilité de changement de chaque caractère.

Toutes les analyses phylogénétiques ont été conduites en utilisant les logiciels informatiques *Phylip* 3.75 *(PHY Logenetic Inference Package)* (Felsenstein et al., 1989) et *Paup* * *version 4. Obêta6* (Phylogenetic Analysis Using Parsimony) (Swofford et al., 1998).

L'analyse de distance a été calculée par la méthode de Kimura à deux paramètres, qui considère les taux de transition (mutations T <-> C et G <-> A) à chaque site, et de transversion (mutations « A ou G » <---> « T ou C ») à chaque site comme différents.

La fiabilité et la robustesse des groupements de séquences (ou des topologies) sont évaluées statistiquement par l'approche de rééchantillonnage (ou bootstrap) sur 10³ et 10⁴ rééchantillonnages.

Les résultats obtenus se présentent sous la forme d'un arbre phylogénétique visualisé par le programme *Treeview* (version 1.6.5), proposant différentes présentations de l'arbre (cladogramme, radial et phylogramme). Il permet également de visualiser les valeurs de bootstrap, à chaque noeud et de déterminer un taxon comme outgroup (séquences du génotype-III).

La traduction en acides aminés du gène delta est effectuée à l'aide du programme *DNAStrider version 1.3.* L'alignement des séquences protéiques est réalisé comme décrit précédemment.

### 8- Analyse génotypique de l'HDV par polymorphisme de restriction (RFLP)

Le génotypage de l'HDV est effectué par PCR-RFLP de la région R0, selon les étapes suivantes :
- Etape 1 : Les produits PCR sont digérés par les deux enzymes de restriction *SmaI* et *XhoI* (New England Biolabs) : 10 µl de produit amplifié sont digérés séparément dans deux tubes par 5 U d'enzymes *Sma*I ou *Xho*I, respectivement à 30°C et 37°C pendant 3 heures dans un volume final de 50 µl en présence du tampon approprié et d'eau stérile. Les produits de digestion sont visualisés sous rayons ultraviolets comme décrit précédemment et les tailles des fragments sont déterminées par comparaison avec un marqueur de taille (50 pb échelle d'ADN, ou les marqueurs V et VI, Life Technologies GibcoBRL).
- Etape 2 : Les échantillons présentant un profil autre que le profil de génotype I sont digérés par une autre enzyme *Sac*II (New England Biolabs) pendant 3 heures à 37°C et les produits de digestion sont visualisés comme à l'étape 1.
- Etape 3 : le génotype du virus est déterminé à partir de l'analyse de la combinaison des profils de restriction *Sma*I, *Xho*I et *Sac*I.

### 9- Algorithme de génotypage de l'HDV par PCR-RFLP

L'algorithme de génotypage de l'HDV par PCR-RFLP comprend au moins deux étapes :
- la première, consiste en la coupure par deux enzymes de restriction, *SmaI* et *XhoI* du fragment R0 amplifié par RT-PCR à partir des ARN extraits des sérums des patients ;
- la seconde pour les patients de « profil non-I », consiste en une coupure de R0 par l'enzyme *SacII* ;
- le séquençage de la région R0 ou de la région codant pour la p24 (ou si nécessaire de la totalité du génome), suivi d'analyses phylogéniques ne seront réalisés qu'en deuxième intention si des profils de restriction inhabituels sont obtenus.

### EXEMPLE 2: MISE EN EVIDENCE DE NOUVEAUX GENOTYPES D'HDV

### 1- Analyse phylogénétique de la région R0

22 échantillons de patients infectés par HBV et HDV ont été analysés. La région R0 a été amplifiée par PCR puis le fragment obtenu a été séquencé en utilisant les amorces 900S et 1280As.

L'étude phylogénétique a été effectuée à partir d'un alignement de séquences de 336 bases de R0 (les régions ambiguës sont éliminées), en y incluant, en plus des 22 séquences étudiées, 15 séquences de référence et 6 séquences R0 d'HDV de Yakoutie (Pt13, 26 (SEQ ID NO:66), 29, 62 (SEQ ID NO:67), 63 et 704). Le nom donné aux séquences correspond à dFr (pour "delta France") suivi du numéro de sérum du patient.

### a) analyse de la distance génétique

L'arbre phylogénétique, obtenu par reconstruction à partir de distances génétiques de la région R0 est présenté à la figure 1.

La topologie de l'arbre individualise les génotypes-I et -III, représentés respectivement par sept et trois séquences nucléotidiques de référence. Les autres séquences de référence sont représentées par les séquences de type II (Japon, Taiwan-3 et les séquences de Yakoutie), et un groupe de deux séquences (TW2b, Miyako) décrites respectivement chacune comme prototype de « sous-types IIB et IIC ».

Cet arbre montre que les séquences virales provenant des 22 échantillons analysés correspondent à deux situations :
- 11 séquences sont affiliées aux séquences de génotype I, à l'exception de la séquence dFr46, qui semble être apparentée à la séquence Us-1 décrite par Makino (Makino et al., 1987) ; toutes ces séquences sont réparties de façon hétérogène au sein du génotype-I.
- les 11 séquences restantes sont très éloignées du génotype-I et du génotype-III. De plus, aucune de ces séquences ne se regroupe directement avec les séquences de type-II (Japon, Taiwan-3, Yakoutie 13, 26, 29, 62, 63, 704) ou au groupe de séquences (TW2b, Miyako) ; ces séquences de référence forment par elles-mêmes deux groupes distincts.

La topologie de l'arbre, obtenu par reconstruction à partir des distances génétiques de la région R0, montre que les molécules nucléiques isolées à partir des divers HDV variants se répartissent au sein de quatre sous-groupes (figure 1) :
- la molécule dFr644 qui apparaît isolée ; elle possède toutefois, avec un groupe de trois molécules (dFr45, dFr2066 et dFr1843), un noeud qui est soutenu pour une valeur de rééchantillonnage de seulement 66,7 %.
- en revanche, la branche unissant les molécules dFr45, dFr2066, dFr1843 est robuste, puisqu'elle est soutenue par une valeur de bootstrap (BV) de 99,9 %.
- un ensemble de cinq molécules : dFr47, dFr910, dFr69, dFr73 et dFr1953 est soutenue par une BV de 100 % et
- une paire de molécules dFr48 et dFr2020, qui est également soutenue par une BV de 100 %.

### b) analyse du maximum de parcimonie

L'arbre phylogénétique, obtenu par reconstruction à partir du maximum de parcimonie de la région R0, est présenté à la figure 2.

L'analyse en maximum de parcimonie soutient la même topologie que l'analyse de la distance génétique. La reconstruction met en évidence l'existence au sein des 11 séquences variantes, des mêmes trois groupes monophylétiques ; par exemple, avec cette approche, le groupe de cinq molécules *dFr47, dFr910, dFr69, dFr73* et *dFrl953* est également soutenu par une BV de 97 % (Figure 2).

Les 11 molécules variantes, les molécules de génotype II et l'ensemble [TW2b, Miyako] semblent dériver d'une branche commune qui pourrait, par comparaison aux séquences de génotype I et de génotype III, individualiser l'ensemble des séquences du génotype II. Cependant, les valeurs de bootstrap soutenant ce branchement sont relativement modestes : 88,5 % en NJ et 64,5 % en MP (rééchantillonnage effectué sur 10⁴ échantillons) comparées à celles du génotype-I (BV = 99.8 %) et du génotype-III (BV= 100 %). De plus, la distance moyenne entre les différents sous-groupes définis au sein des 11 HDV variants ou entre ces variants et les séquences de génotype-II, apparaît plus élevée qu'entre l'ensemble des isolats du génotype-I ou au sein des trois molécules définissant le génotype-III.

L'ensemble de ces résultats mettent en valeur la caractérisation de nouveaux génotypes d'HDV.

### 2 - Analyse phylogénétique de la totalité des génomes

### a) Reconstruction du génome complet à partir de fragments amplifiés

Afin d'étudier le génome complet de ces variants et, dans le but de préciser leur affiliation, plusieurs régions du génome HDV ont été amplifiées (Tableau II) à partir de 6 échantillons incluant au moins un membre de chacun des 4 sous-groupes et trois représentants du groupe majoritaire ont été sélectionnés : *dFr45, dFr47, dFr48, dFr73, dFr644 et dFr910.*

De manière plus précise, les fragments suivants ont été amplifiés par PCR (Tableau IV) :
- des fragments de 850 pb (R'1) et 1050 pb (R'2) chevauchants à leurs extrémités pour *dFr45, dFr47, dFr48 et dFr73,*
- deux fragments chevauchants de 960 pb et de 1250 pb pour *dFr644,* et
- deux fragments de 1400 pb et 650 pb pour *dFr910.*

Toutes ces régions génomiques amplifiées ont été clonées dans un vecteur pCRII™ (Tableau VI). Deux clones correspondant à chacun des fragments amplifiés ont été séquencés. La reconstruction de séquences complètes consensus d'ADNc HDV a été effectuée après alignement des régions chevauchantes et alignement avec les séquences de référence.

**Tableau VI : Clones de pcRII contenant les différents inserts**

| | R0 | R'1 | R'2 | G910 | R1 |
|---|---|---|---|---|---|
| dFr45 | - | dFr45R'1 clone 2 | dFr45R2 clone 8 | - | - |
| | | dFr45R'1 clone 4 | dFr45R2 clone 10 | | |
| dFr47 | - | dFr47R'1 clone 13 | dFr47R2 clone 19 | - | - |
| | | dFr47R'1 clone 16 | dFr47R2 clone 22 | | |
| dFr48 | - | dFr48R'1 clone 23 | dFr48R2 clone 19 | - | - |
| | | dFr48R'1 clone 28 | dFr48R2 clone 22 | | |
| dFr73 | - | dFr73R'1 clone 36 | DFr73R2 clone 29 | - | - |
| | | dFr73R'1 clone 39 | dFr48R2 clone 33 | | |
| dFr644 | - | - | - | - | 644R1 clone 4 |
| | | | | | 644R1 clone 8 |
| dFr910 | 910R0-clone 4 | - | - | R910 clone 29 | 910R1 clone 4 |
| | 910R0-clone 4 | | | R910 clone 31 | 910R1 clone 5 |

### b) Analyse de six nouvelles séquences génomiques complètes d'HDV, d'origine africaine.

### b₁) Caractéristiques cliniques des 6 patients

Cinq patients sont originaires d'Afrique de l'Ouest, et un patient a séjourné au Cameroun. Au moment du prélèvement, ces patients résidaient en région parisienne depuis au moins deux ans. Tous ces patients étaient atteints d'hépatite sévère et les données cliniques sont résumées dans la figure 3.

### b₂) l'organisation génomique des nouvelles séquences d'HDV

L'analyse comparative des régions R0 de 22 patients infectés par HDV et HBV avec celles disponibles dans les bases de données a mis en évidence la grande diversité génétique du génome viral de l'HDV.

La taille des génomes complets est différente pour les six séquences des six isolats d'HDV d'origine africaine, ce qui confirme la variabilité de l'HDV :
- le génome viral des isolats *dFr910, dFr47* et *dFr73* qui comprend 1697 nucléotides est le plus long jamais décrit pour HDV.
- le génome de l'isolat *dFr45* apparaît parmi les plus petits (1672 nt), et
- les séquences génomiques des virus *dFr644* et *dFr48* sont respectivement de 1680 nt et de 1687 nt.

L'analyse après l'alignement des différentes séquences étudiées, révèle un degré élevé de conservation dans les régions du génome HDV correspondant aux ribozymes responsables des clivages des ARN génomiques et antigénomiques. De même, le cadre de lecture codant pour l'antigène delta est retrouvé sur le brin antigénomique. Un codon tryptophane (UGG) est le seul à être caractérisé pour deux séquences *(dFr47, dFr910)* et une ambiguïté (G/A) retrouvée pour les quatre autres séquences indique que la petite et la grande protéine delta sont très probablement synthétisées. Les régions variables comprennent la portion non codante ainsi que les extrémités 5' et 3' du gène *LHD.* De façon remarquable, une insertion de 7 nucléotides existe dans la séquence *dFr48.* Cette insertion est présente dans une boucle correspondant à l'une des extrémités du génome dans sa forme pseudo double brin (à la position 797 de la séquence de référence Italie (Wang et al., 1987).

### c) Comparaison des six séquences HDV d'origine africaine avec les séquences représentatives des différents génotypes.

La comparaison des six nouvelles molécules avec les molécules connues, représentatives des trois génotypes connus, indique une similarité en nucléotides se situant entre 71,7 % (*dFr45 versus* Liban) et 80,0 % (*dFr73 versus* Yakoutie p26) au regard des molécules de génotypes I, II, et des molécules TW2b et Miyako. En effet, pour chacun des six isolats, la moyenne de similarité en nucléotides est de l'ordre de 73,3 % à 74,6 % avec les molécules du génotype I, de 74,5 % à 78,8 % avec celles du génotype II et de l'ordre de 74,6 % à 77,8 % avec les molécules TW2b/Miyako. En revanche, la similarité nucléotidique avec l'isolat du Pérou (génotype-III), n'est que de 63,9 % à 66,0 % confirmant l'éloignement particulier de cette molécule (Tableau VII). De plus, lorsque l'on compare, entre elles les six molécules correspondant à ces génomes complets et définissant les six variants *dFr45, dFr47, dFr48, dFr73, dFr644* et *dFr910,* seul le groupe de molécules *dFr73, dFr910* et *dFr47* présente une similarité de séquence de l'ordre de 90 %. Les molécules *dFr45, dFr48,* et *dFr644* sont aussi éloignées l'une de l'autre que des génotypes I, II, des séquences TW2b / Miyako et du groupe de molécules *dFr73, dFr910* et *dFr47* (de l'ordre de 73,2 % à 78 %) (Tableau VIII).

**Tableau VII : Pourcentage de similarité des séquences complètes HDV Africaines avec les différents génotypes connus (Calcul de la moyenne).**

| Isolats HDV* | Type I | Type II | TW2b/Miyako | Type III |
|---|---|---|---|---|
| *dFr45* | **73,3** | **74,5** | **74,6** | **66** |
| | *71,7 - 74,6* | *73,2 - 75,5* | | |
| *dFr47* | **74,2** | **78,6** | **77,4** | **65,5** |
| | *73,0 - 75,0* | *78,2 - 79,9* | | |
| *dFr48* | **73,3** | **77,1** | **75,5** | **65,4** |
| | *72,0 - 74,0* | *76,6 - 77,7* | *74,4 - 76,6* | |
| *dFr73* | **74,1** | **78,8** | **77,8** | **65,9** |
| | *73,0 - 75,0* | *77,7 - 80,0* | *77,5 - 78,0* | |
| *dFr644* | **73,6** | **76,8** | **77,0** | **63,9** |
| | *72,2 - 74,6* | *76, 2 - 77,2* | *76,9 - 77,2* | |
| *dFr910* | **74,6** | **77,9** | **77,2** | **64,6** |
| | *73,0 - 75,8* | *77,0 - 78,6* | *77,0 - 77,5* | |

| | | | | |
|---|---|---|---|---|
| * Les isolats HDV de référence correspondent aux génomes complets étudiés à l'exemple 2.1. | | | | |

**Tableau VIII : Pourcentage de similarité des nouvelles molécules HDV entre elles.**

| | *dFr47* | *dFr48* | *dFr73* | *dFr644* | *dFr9l0* |
|---|---|---|---|---|---|
| *dFr45* | 74,8 | 73,2 | 75 | 78 | 74,7 |
| *dFr47* | | 77,1 | **90** | 76,3 | **89** |
| *dFr48* | | | 77,7 | 75,5 | 76,1 |
| *dFr73* | | | | 76,3 | **89** |
| *dFr644* | | | | | 76,1 |

### d) Analyse phylogénétique des six molécules HDV d'origine africaine et des molécules représentatives des différents génotypes

L'analyse phylogénétique a été menée sur les six séquences complètes d'origine africaine, seize séquences de référence et 2 séquences de Yakoutie (Pt26 et Pt62). La Figure 4 illustre les résultats obtenus par analyse de distance. L'arbre phylogénétique reconstruit par *"Neighbor-Joining"* (NJ) montre qu'aucune des six séquences étudiées (*dFr45, dFr47, dFr48, dFr73, dFr644* et *dFr9l0*) ne se regroupe avec les séquences de référence du génotype I ou du génotype III. L'affiliation de ces séquences africaines aux séquences du génotypes II (aux séquences TW2b et Miyako décrites respectivement comme les sous-types IIB et IIC) n'est pas soutenue, par des valeurs de bootstrap élevées (<70 %) (Wu et al., 1998). De plus, les séquences TW2b et Miyako apparaissent former un groupe distinct et monophylétique avec une BV de 100 %. Ces deux séquences semblent constituer par elles-mêmes un "clade" représentant un génotype différent du type II.

Dans les analyses de distance, les six séquences africaines sont subdivisées en 3 sous-groupes distincts (supportés par des BV supérieures à 90,3 % pour 10⁴ rééchantillonnages). Les séquences *dFr47, dFr73* et *dFr910* constituent un groupe dont la branche repose sur une valeur de bootstrap de 100 %. Pour appuyer ces résultats, l'étude du maximum de parcimonie a été conduite sur le même jeu de séquences (Figure 5). En enracinant, l'arbre de façon artificielle grâce à la séquence 'Peru-1', on individualise comme dans toutes les analyses menées précédemment l'ensemble des séquences du génotype-I (BV = 100 %). La topologie des autres séquences soutient la répartition des isolats africains et asiatiques en plusieurs groupes ; ceci montre l'intérêt de l'utilisation de la région R0. Le génotype-II regroupe les séquences de Yakoutie, Taiwan-3 et Japan avec une BV de 99,9 % sur 10⁴ rééchantillonnages. De même, l'individualisation de TW2b et Miyako se confirme (BV = 100 %). Enfin, les séquences africaines indiquent l'existence d'au moins 3 sous-groupes. La monophylie des séquences *dFr47, dFr73, dFr9l0* (BV =100 %) soutient l'affiliation de ces séquences dans un sous-groupe. En revanche, la séquence *dFr48,* qui possède avec les isolats du groupe précédant (*dFr910, dFr47, dFr73*) une similarité nucléotidique respective de 76,1, 77,1 et 77,7 % se regroupe avec ces séquences seulement dans 55,4 % des rééchantillonages, suggérant son individualisation possible. Bien qu'apparaissant distantes l'une de l'autre, le groupement *dFr45* et *dFr644,* s'observe avec une BV élevée (NJ = 96.5 / MP = 88,6) dans le contexte étudié.

Par conséquent, à la fois les analyses phylogénétiques des régions R0 et des séquences complètes des séquences africaines indiquent que les groupes diffèrent les uns des autres et pourraient constituer trois (voir quatre) génotypes distincts ; ces résultats mettent ainsi en évidence l'existence d'au moins sept génotypes d'HDV.

### 3- Analyse de la séquence en acides aminés (aa) de l'antigène delta (Ag-HD).

L'Ag-HD est représenté par les deux formes p24 (sHD) et p27 (LHD) de la protéine delta. La séquence protéique de 1 à 194-195 acides aminés correspond à la petite protéine delta (sHD) ou forme p24. La grande protéine delta (LHD) ou forme p27 possède la même extrémité amino-terminale et une extension de 19 à 20 acides aminés à son extrémité carboxy-terminale.

L'alignement de la séquence de l'antigène HD des six séquences africaines avec les séquences des protéines HD connues est présenté à la figure 6.

L'analyse des séquences montre que les six isolats d'origine africaine ont une identité en amino-acides de l'ordre de 69 à 77 % avec les séquences du génotype I, de 71 à 79 % avec les isolats du génotype II, de 72 à 78 % avec les séquences TW2b / Miyako et de 63 % avec l'isolat du Pérou (génotype III).

La taille des protéines correspondant aux nouveaux isolats varie entre 213 et 214 acides aminés. Toutes ces protéines ont le même profil d'hydrophobicité. La forme p24 possède 2 petites régions hydrophobes, l'une située aux alentours des acides aminés 50-60 (entre le site de polymérisation et le SLN) et l'autre entre les positions 160 et 172 (en regard d'un motif extrêmement conservé). Deux autres domaines sont bien conservés entre les différents génotypes : il s'agit du domaine de fixation à l'ARN et du domaine de localisation nucléaire. A l'image de ce qui a été décrit dans la littérature, l'extrémité carboxy-terminale de la protéine delta (entre les acides aminés 195 et 215) constitue une région hypervariable. Deux acides aminés seulement sur les 19-20 sont conservés. Il s'agit de la cystéine (C) correspondant au site de farnésylation de la forme large de la protéine HD et de la glycine (G) carboxy-terminale. De plus, on retrouve les séquences signatures spécifiques des isolats du même génotype, par exemple, les 19 acides aminés spécifiques de la grande protéine du génotype I ou les 20 acides aminés du génotype-III.

En revanche, pour les séquences protéiques des isolats d'origine africaine, des isolats du génotype Il et de TW2b/Miyako, l'extrémité carboxy-terminale semble subdivisée en deux domaines. Le domaine variable est représenté par les acides aminés 197 à 205 et le domaine conservé allant des acides aminés 206 à 215 (RLPLLECTPQ) (Figure 5).

### 4- Définition de 7 clades d'HDV

L'analyse des séquences complètes des six isolats d'Afrique permet de définir sept clades d'HDV correspondants aux génotypes suivants (Tableau IX) :

**Tableau IX : Correspondance Clade/Génotype**

| Clade | Génotype | Isolats |
|---|---|---|
| 1 | I | Italie, W5, W15, Us 1, Us2, Liban, Ethiopie Somalie, Ile de Nauru, Chine, Cagliari, Canada... |
| 2 | IIA | Japon, Taiwan3, Yakutie26, Yakutie62 |
| 3 | III | Pérou1 |
| 4 | IIB, IIC | TW2b, Miyako |
| 5 | ? | dFr910, dFr73, dFr47 |
| 6 | ? | dFr 48 |
| 7 | ? | dFr45, dFr644 |

### EXEMPLE 3: METHODE DE GENOTYPAGE DE HDV-1 à HDV-7 par PCR-RFLP

Le génotypage est effectué selon le protocole décrit à l'exemple 1.8.

### 1- Manque de sensibilité de la PCR 6A/6S

Initialement, trois patients Ag-HBs positifs, ont posé un problème diagnostique d'infection delta. En effet chez ces patients on observe une hépatite sévère associée à la présence d'IgM anti-HDV mais une absence de réplication d'HDV par RT-PCR à l'aide des amorces 6A-6S décrites dans Deny et al. (1991, 1993, 1994, précités) pour le diagnostic en routine de l'infection par HDV. La PCR 6A/6S amplifie un fragment d'ADNc de 234 pb correspondant à la partie de l'extrémité carboxyterminale du gène LHD (positions 904 à 1141 sur le génome viral).

Les ARN extraits du sérum de ces mêmes patients ont été réamplifiés à l'aide du couple d'amorces 900S et 1280AS définissant la région R0.

Les résultats obtenus à partir des échantillons de ces trois patients ont mis en évidence la présence reproductible d'une bande de 400 pb (R0) avec les amorces 900S et 1280AS, alors que la PCR 6A-6S restait négative.

Ces résultats ont été confirmés sur une série d'échantillons de sérum de patients qui ont été analysés en parallèle avec les couples d'amorces 6A-6S et 900S-1280AS. Sur 286 échantillons, 14 sont positifs uniquement avec la PCR R0.

Ces résultats démontrent une plus grande spécificité et une meilleure sensibilité des amorces 900S et 1280AS, en comparaison avec les amorces 6S et 6A, pour la détection de l'ARN d'HDV dans le sérum des patients infectés.

### 2- Profils de restriction attendus pour HDV-1 à HDV-7

Les méthodes classiquement utilisées de PCR-*RFLP* (Wu et. al., 1995a ; Wu et al., 1995b ; Casey et al., 1996) permettent la distinction de trois différents génotypes delta. L'utilisation de l'enzyme de restriction *SmaI* ne différencie pas tous les génotype-I, -IIA, -IIB reconnus à ce jour et l'enzyme *XhoI* a été utilisée pour différencier le «sous-type IIA» du «sous-type IIB» (Wu et al., 1995b).

L'association des deux enzymes *SmaI* et *XhoI* dans une première étape révèle sept profils distincts (de P1 à P7) (Tableau X). Ces sept profils ne se superposent pas exactement aux sept clades (HDV-1 à HDV-7). En conséquence, les échantillons de profil «non-P1» sont coupés dans une deuxième étape par l'enzyme *SacII,* aboutissant ainsi à l'obtention, de façon combinée, de dix profils delta distincts (de D1 à D10) (Tableau XI) pouvant être rattachés spécifiquement aux différents clades décrits, grâce aux analyses de phylogénie.

**Tableau X : Profils de restriction coupure de la Région R0, par les enzymes SmaI et XhoI.**

| **ETAPE 1** Génotypes décrits | Fragments *Sma*I Taille (pb) | Profil *SmaI* | Fragments *Xho*I Taille (pb) | Profil *XhoI* | Profil Combiné *SmaI- XhoI* | |
|---|---|---|---|---|---|---|
| I | 220,179 | *S1* | 383,16 | *X1* | *S1 X1* | **P1** |
| IIA | 397 | *S2* | 303,78,16 | *X2* | *S2 X2* | **P2** |
| IIB | 397 | *S2* | 319,79 | *X3* | *S2 X3* | **P3** |
| IIC (Miyako) | 397 | *S2* | 157,162,79 | *X4* | *S2 X4* | **P4** |
| III | 298,107 | *S3* | 405 | *X5* | *S3 X5* | **P5** |
| | | | | | | |
| II Yakutie | 178,117,110 | *S4* | 303,78,16 | *X2* | *S4 X2* | **P6** |
| *dFr45* | 217,179 | *S1* | 303,78,16 | *X2* | *S1 X2* | **P7** |
| *dFr644* | 217,179 | *S1* | 303,78,16 | *X2* | *S1 X2* | **P7** |
| *dFr47, 73,* | 179,111,107 | *S4* | 303,78,16 | *X2* | *S4 X2* | **P6** |
| *910* | | | | | | |
| *dFr48* | 397 | *S2* | 303,78,16 | *X2* | *S2 X2* | **P2** |

**Tableau XI : Profils de restriction attendus après coupure de la Région R0, par les enzymes SmaI, XhoI et SacII.**

| **ETAPE 2** Génotypes décrits | Fragments *Sac*II Taille (pb) | Profil *Sac*II | Profil Combiné *SmaI- XhoI* / *SacII* | |
|---|---|---|---|---|
| I | 362,38 | *Sc1* | *S1 X1 Sc1* | **D1** |
| IIA | 266,92,38 | *Sc2* | *S2 X2 Sc2* | **D2** |
| IIB | 268,130 | *Sc3* | *S2 X3 Sc3* | **D3** |
| IIC (Miyako) | 268,130 | *Sc3* | *S2 X4 Sc3* | **D4** |
| III | 405 | *Sc4* | *S3 X5 Sc4* | **D5** |
| | | | | |
| II Yakutie | 266,92,38 | *Sc2* | *S4 X2 Sc2* | **D6** |
| *dFr45* | 268,130 | *Sc3* | *S1 X2 Sc3* | **D7** |
| *dFr644* | 397 | *Sc4* | *S1 X2 Sc4* | **D8** |
| *dFr47, 73, 910* | 268,130 | *Sc3* | *S4 X2 Sc3* | **D9** |
| *dFr48* | 268,130 | *Sc3* | *S2 X2 Sc3* | **D10** |

### 3- Génotypage des échantillons de patients par PCR-RFLP

A partir de l'analyse par PCR-RFLP d'échantillons (plus de 50) :
- aucun génotype-II ou -III n'a été retrouvé.
- 89,7 % des patients présentaient un profil D1 (génotype-I) et 10,3% un profil « non-I »
- deux nouveaux profils *XhoI* (X6 et X7) entraînant trois combinaisons nouvelles (D11, D12 et D13) supplémentaires ont été détectés (Tableaux XII et XIII).

**Tableau XII : Nouveaux profils de restriction XhoI obtenus chez cinq patients originaires d'Afrique de l'Ouest**

| **ETAPE 1** PATIENTS | Fragments *Sma*I Taille (pb) | Profil *SmaI* | Fragments *Xho*I Taille (pb) | Profil *XhoI* | Profil Combiné *SmaI-XhoI* | |
|---|---|---|---|---|---|---|
| dFr1843 | 218, 179 | *S1* | 303, 78, 16 | *X2* | *S1 X2* | **P7** |
| dFr1953 | 218, 179 | *S1* | 303, 78, 16 | *X2* | *S1 X2* | **P7** |
| dFr2020 | 392 | *S1* | 303, 73, 16 | *X2* | *S2 X2* | **P2** |
| dFr2088 | 220, 179 | *S1* | 242, 171, 16 | *X6* | *S1 X6* | **P8** |
| dFr2066 | 217, 179 | *S1* | 237, 66, 16 | *X7* | *S1 X7* | **P9** |

**Tableau XIII : Nouveaux profils de restriction XhoI, SmaI, SacII, obtenus chez cinq patients originaires d'Afrique de l'Ouest**

| **ETAPE 2** PATIENTS | Fragments *Sac*II Taille (pb) | Profil *SacII* | Profil Combiné *SmaI-XhoI* / *SacII* | |
|---|---|---|---|---|
| dFr1843 | 267, 130 | *Sc3* | *S1 X2 Sc3* | **D7** |
| dFr1953 | 267,92,38 | *Sc2* | *S1 X2 Sc2* | **D11** |
| dFr2020 | 262, 130 | *Sc3* | *S2 X2 Sc3* | **D10** |
| dFr2088 | 396 | *Sc4* | *S1 X6 Sc4* | **D12** |
| dFr2066 | 396 | *Sc4* | *S1 X7 Sc4* | **D13** |

La correspondance entre les profils combinés et les génotypes identifiés par l'anlyse phylogénétique est présentée dans le Tableau XIV.

**Tableau XIV : Résumé des différents résultats basés sur les analyses phylogénétiques et les différents profils correspondants**

| **Clades** | **Génotypes** | **Isolats** | **Profils combinés** (*Sma*I*-Xho*I / *Sac*II) |
|---|---|---|---|
| **HDV-1** | I | Italie | **D1A** |
| | | dFr2088 | **D1B** |
| **HDV-2** | IIA | Japon | **D2A** |
| | | isolats de Yakoutie | **D2B** |
| **HDV-3** | III | Pérou1 | **D3** |
| **HDV-4** | IIB | TW2b | **D4A** |
| | IIC | Miyako | **D4B** |
| **HDV-5** | V | dFr47, dFr73 et dFr910 | **D5A** |
| | | dFr1953 | **D5B** |
| **HDV-6** | VI | dFr48, dFr2020 | **D6** |
| **HDV-7** | VII | dFr45, dFr1843 | **D7A** |
| | | dFr2066 | **D7B** |
| | | dFr644 | **D7C** |

### RÉFÉRENCES BIBLIOGRAPHIQUES

- Casey J.L. et al., Proc. Natl. Acad. Sci. USA, 1993a, 90, 9016-20.
- Casey J.L. et al., J. Infect. Dis., 1996b, 174, 920-6.
- Chang F.L. et al., Proc. Natl. Acad. Sci. USA, 1991, 88, 8490-8494.
- Chao Y.C. et al., Hepatology, 1991b, 13, 345-52.
- Deny P. et al., Res. Virol., 1994, 145, 287-95.
- Deny P. et al., J. Med. Virol., 1993, 39, 214-8.
- Deny P. et al., J. Gen. Virol., 1991, 72, 735-9.
- Felsenstein J. et al., Cladistics, 1989, 5, 164-166.
- Gaeta G.B. et al., Hepatology, 2000, 32, 824-7.
- Glenn J.S. et al., Science, 1992, 256, 1331-3.
- Hwang S. et al., Virology, 1993a, 193, 924-931.
- Imazeki F. et al., J. Virol., 1990, 64, 5594-5599.
- Imazeki F. et al., Nucl. Acid. Res., 1991, 19, 5439-5440.
- Lai M.M.C. et al., S. Hadziyannis, J. Taylor et F. Bonino (ed.), Hepatitis delta virus. Molecular biology, Pathogenis, and Clinical aspects, 1993, 382, 21-27. Wiley-Liss, New York.
- Lee C.M. et al., Virology, 1992, 188, 265-273.
- Lee C.M. et al., J. Med. Virol., 1996b, 49, 145-54.
- Makino S. et al., Nature, 1987, 329, 343-6.
- Nakano T. et al., J. Gen. Virol., 2001, 82, 2183-2189.
- Niro G.A. et al., J. Hepatol., 1999, 30, 564-9.
- Niro G.A. et al., Hepatology, 1997, 25, 728-34.
- Roingeard P. et al., Clin. Infect. Dis., 1992, 14, 510-14.
- Saitou N. et al., Mol. Biol. Evol., 1987, 4, 406-25.
- Sakugawa H. et al., J. Med. Virol., 1999, 58, 366-72.
- Shakil A.O. et al., Virology, 1997, 234, 160-7.
- Swofford D. et al., PAUP*: Phylogenetic Analysis Using Parsimony (and other methods), version 4.0d64.
- Thompson J.D. et al., Nuc. Acid. Res., 1994, 22, 4673-80.
- Wang K.S. et al., Nature, 1986, 323, 508-514.
- Wang K.S. et al., Nature, 1987, 328, 456.
- Wu J.C. et al., Hepatology, 1995a, 22, 1656-60.
- Wu J.C. et al., J. Gen. Virol., 1998, 79, 1105-13.
- Wu J.C. et al., Lancet, 1995b, 346, 939-41.

### SEQUENCE LISTING

<110> ASSISTANCE PUBLIQUE-HOPITAUX DE PARIS
   DENY Paul
   RADJEF Nadjia
   HUC-ANAIS Patricia
<120> .
<130> S1020FR17
<140>
   <141>
<160> 67
<170> PatentIn ver. 2.1
<210> 1
   <211> 1672
   <212> DNA
   <213> hepatitis D virus
<220>
   <221> source
   <222> (1)..(1672)
   <223> complete genomic sequence of dFr45
<400> 1
   atgagccaag tccgacgaag agtccggaga tgggagagga gagagacccc cgaggaggca 60
   aagtcgagag ggcggagaca ctctgaggag aggactccca agaggacaag aaggactcaa 120
   gatccgggaa cgaatcccca aaacgccggt aaagctcttg gaatgaggaa aacgggcggg 180
   atggtagaaa agagcgagcc tcccgattac gagattcccc tgaacttatt ggatctggag 240
   actccgacct taaggggtca aaggtaggac agggaggcaa tccaccagga gatttcggac 300
   aactcacctc cagaggaccc cttcagcgaa cagaagagcc tgtcccggtc gaattgtccc 360
   gtagcgatag gaggagatgc taggagtagg gagagaccgg tgcgagagag gcaagacaag 420
   agagcagcgg ggctagcgag tggatgttcc gccccccgtt ggctccgagt gaggcttatc 480
   ccggggaatt tggctawctt ccccaactag ccggtcccgg atccccttcc aratgaacgg 540
   ggggactccg gcctctcggg gaatcggcgg ctccatggtg aacccccgca gccccccact 600
   ccacactcct tccccccgcg gggcccccca tccaaagatg gaactccacc cctcagggtt 660
   cgcyatccta ccctttcctt acctttggcc ggcatggtcc cagcctcctc gctggcgccg 720
   gctgggcaac attccgaggg gaccgtccct cggcaatggc gaatgggacc caagacctcc 780
   aagattccca gagagaatct ggaggtgact gggcactccc tttgccatcc gagtggacgt 840
   tcgtcctcct tcggatgccc aggtcggacc acggggaggt ggagatgcca tgccgacccg 900
   aagaggaaag aaggattcgg acgcaaacct gtgagtggaa gttcgctctt tattgggggg 960
   tacactcgag gagtggaagg cggggagggg gggtcggact aggtccctat ggaaactgtc 1020
   ggtctcctcg gatgtcgagt ccctctcccg ttctggagaa gggggactcc gggacaccta 1080
   gcagttgagg aacgaagccg cccccgggcg ctcccctcgg tggtccctcg ggagggttca 1140
   catccccaac ccgcgggccg gctattcttc ttgctctttg ctcgtcatcg gcggtcaact 1200
   tcctgagttc ctcttcttcc tccttgctga gggactttcc tcctgcggac agctgcttct 1260
   tcttgttctc gagggccttc cttcgtcggt gagcccgtct ctcctcgtcg gtgaagcctc 1320
   ccttgttact ctttttccct ggtccggagt cgacctccat ccgatctgtt cggggtctct 1380
   tcgccggggg agctccctcc ccagtcccga cctttccaat tattcctttg atgtttccca 1440
   gccagggatt gtcgtcctca agtttcttga tggtcttctt tgtcttccgg agcctcctct 1500
   cgagttcttc cgcgtcttcc cttgctttgw cccacttcga aagggtctcc tcccttccct 1560
   tccgggatct cttcgtatcg gcgtggctca tctcggcaga ggcgggcgat cctcagttct 1620
   cttactcttt tcttgaaaga ggagactgct ggacgctccg ccgtgtccga gc 1672
<210> 2
   <211> 642
   <212> DNA
   <213> hepatitis D virus
<220>
   <221> CDS
   <222> (1)..(642)

## Revendications

1. Molécules d'acide nucléique isolées, **caractérisées en ce qu'**elles sont sélectionnées dans le groupe constitué par :
- le génome complet du variant d'HDV dénommé dFr48 qui présente la séquence SEQ ID NO: 21, et
- le génome d'un HDV qui présente une divergence génétique ≤ 15 % avec la séquence SEQ ID NO: 21.

2. Molécules d'acide nucléique, isolées **caractérisées en ce qu'**elles comprennent au moins l'un des fragments des séquences d'un HDV variant selon la revendication 1, sélectionnés dans le groupe constitué par :
a) le fragments R0 qui présente la séquence SEQ ID NO: 50,
b) le fragment R1 qui s'étend des positions 307 à 1289 du génome d'HDV,
c) le fragment R2 qui s'étend des positions 889 à 328 du génome d'HDV,
d) le fragment R3 qui s'étend des positions 1486 à 452 du génome d'HDV,
e) le fragment R'1 qui s'étend des positions 305 à 1161 du génome d'HDV,
f) le fragment R'2 qui s'étend des positions 984 à 331 du génome d'HDV,
g) le fragment R644 qui s'étend des positions 889 à 446 du génome d'HDV,
h) le fragment G910 qui s'étend des positions 1206 à 929 du génome d'HDV,
i) le fragment p910 qui s'étend des positions 553 à 1550 du génome d'HDV,
j) l'ADNc codant pour la protéine sHD, de séquence SEQ ID NO: 24 et
k) l'ADNc codant pour la protéine LHD, de séquences SEQ ID NO: 22.

3. Méthode de détection d'un HDV variant tel que défini à la revendication 1, par hybridation et/ou amplification, réalisée à partir d'un échantillon biologique, laquelle méthode est **caractérisée en ce qu'**elle comprend :
(1) une étape d'extraction de l'acide nucléique à détecter, appartenant au génome du virus, éventuellement présent dans l'échantillon biologique,
(2) la réalisation d'au moins une amplification génique à l'aide d'une paire d'amorces sélectionnée dans le groupe constitué par les amorces aptes à amplifier l'une des régions suivantes de l'ARN génomique d'HDV : R0, R1, R2, R3, R644, G910, p910, R'1 et R'2 et
(3) l'analyse du produit amplifié par comparaison avec la molécule de séquence SEQ ID NO: 21.

4. Méthode de détection selon la revendication 3, **caractérisée en ce que** l'étape (3) d'analyse peut être mise en oeuvre par restriction, séquençage ou hybridation.

5. Méthode selon la revendication 3 ou la revendication 4, **caractérisée en ce que** les amorces spécifiques pour l'amplification des régions R0, R1, R2, R3, R644, G910, p910, R'1 et R'2, mises en oeuvre à l'étape (2) sont sélectionnées dans le groupe constitué par :
- les amorces 900S (SEQ ID NO:33) et 1280AS (SEQ ID NO:34), pour l'amplification de RO (environ 400 pb),
- les amorces 320S (SEQ ID NO:39) et 1280AS (SEQ ID NO:34), pour l'amplification du fragment R1 (environ 960 pb),
- les amorces 900S (SEQ ID NO:33) et 320AS (SEQ ID NO:45), pour l'amplification de R2 (environ 1100 pb), qui contient le gène sHD correspondant aux positions 1598-950,
- les amorces 1480S (SEQ ID NO:46 ) et 440AS (SEQ ID NO:47), pour l'amplification de R3 (environ 650 pb),
- les amorces 318S (SEQ ID NO:35) et 1150AS (SEQ ID NO:36), pour l'amplification de R'1 (environ 850 pb),
- les amorces 960S (SEQ ID NO:37) et 345AS (SEQ ID NO:38), pour l'amplification de R'2 (environ 1050 pb).

6. Méthode de détection et de génotypage d'HDV à partir d'un échantillon biologique, laquelle méthode est **caractérisée en ce qu'**elle comprend :
(a) une étape d'extraction de l'acide nucléique appartenant au génome du virus HDV,
(b) une étape d'amplification de la région R0 délimitée par les positions 889 à 1289 du génome HDV,
(c) un premier traitement des molécules d'acides nucléiques amplifiées par les enzymes de restriction *Sma*I et *Xho*I, pour produire un premier ensemble de fragments de restriction,
(d) un deuxième traitement des molécules d'acides nucléiques par l'enzyme de restriction SacII, pour produire un deuxième ensemble de fragments de restriction,
(e) l'analyse combinée des deux ensembles de fragments de restriction produits par RFLP (*Restriction Fragment Length Polymorphism*), de manière à détecter la présence de l'HDV dFr48 présent dans ledit échantillon biologique.

7. Méthode selon la revendication 6, **caractérisée en ce que** l'étape (b) d'amplification des molécules d'acide nucléique dudit échantillon par RT-PCR est avantageusement mise en oeuvre avec les amorces 900S (SEQ ID NO: 33) et 1280AS (SEQ ID NO:34).

8. Méthode selon la revendication 6 ou la revendication 7, **caractérisée en ce qu'**elle comprend en outre :
(f) l'amplification des molécules d'acide nucléique dudit échantillon par RT-PCR avec les amorces 900S (SEQ ID NO:33) et 320AS (SEQ ID NO: 45) de manière à amplifier la région R2 et
(g) le séquençage direct de la région R2 amplifiée et la comparaison avec la molécule d'ARN de séquence SEQ ID NO: 21.

9. Vecteur recombinant, notamment un plasmide, comprenant un insert constitué par une molécule d'acide nucléique selon l'une quelconque des revendications 1 ou 2.

10. Cellule transformée par une molécule d'acide nucléique selon l'une quelconque des revendications 1 ou 2.

11. Produits de traduction codés par la molécule d'ARN de séquence SEQ ID NO: 21, par ses séquences complémentaires sens ou anti-sens.

12. Protéines, **caractérisées en ce qu'**elles sont codées par l'une des molécules d'acide nucléique selon l'une quelconque des revendications 1 ou 2.

13. Protéine selon la revendication 12, **caractérisée en ce qu'**elle est sélectionnée dans le groupe constitué par :
- la protéine LHD de dFr48 qui présente la séquence SEQ ID NO: 23, et
- la protéine sHD de dFr48 qui présente la séquence SEQ ID NO: 25.

14. Peptide, **caractérisé en ce qu'**il est constitué par un fragment d'une protéine selon la revendication 12 ou la revendication 13, sélectionné dans le groupe constitué par :
- le peptide A constitué par les 19 acides aminés de l'extrémité carboxy-terminale de la séquence SEQ ID NO: 23,
- le peptide C de séquence SEQ ID NO: 64.

15. Utilisation d'une molécule d'acide nucléique selon l'une quelconque des revendications 1 ou 3 ou d'une protéine selon l'une quelconque des revendications 11 à 14 pour la préparation d'un kit de détection et de génotypage d'un HDV.

## Claims

1. Isolated nucleic acid molecules, **characterised in that** they are selected from the group consisting of:
- the complete genome of the HDV variant referred to as dFr48, which has the sequence SEQ ID NO:21, and
- the genome of an HDV variant which has a genetic divergence of ≤ 15% from sequence SEQ ID NO:21.

2. Isolated nucleic acid molecules, **characterised in that** they include at least one of the fragments of the sequences of a variant HDV according to claim 1, selected from the group consisting of:
a) the R0 fragment which has the sequence SEQ ID NO:50,
b) the R1 fragment which extends from positions 307 to 1289 of the HDV genome
c) the R2 fragment which extends from positions 889 to 328 of the HDV genome,
d) the R3 fragment which extends from positions 1486 to 452 of the HDV genome,
e) the R'1 fragment which extends from positions 305 to 1161 of the HDV genome,
f) the R'2 fragment which extends from positions 984 to 337. of the HDV genome,
g) the R644 fragment which extends from positions 889 to 446 of the HDV genome,
h) the G910 fragment which extends from positions 1206 to 929 of the HDV genome,
i) the p910 fragment which extends from positions 553 to 1550 of the HDV genome,
j) the cDNA encoding the sHD protein, of sequence SEQ ID NO:24, and
k) the cDNA encoding the LHD protein, of sequence SEQ ID NO:22.

3. Method of detection of a variant HDV as defined in claim 1, by hybridisation and/or amplification, carried out from a biological sample, which method is **characterised in that** it comprises:
(1) a step of extracting the nucleic acid to be detected, belonging to the genome of the virus possibly present in the biological sample,
(2) performing at least one gene amplification using a pair of primers selected from the group consisting of the primers capable of amplifying one of the following regions of the HDV genomic RNA: R0, R1, R2, R3, R644, G910, p910, R'1 and R'2, and
(3) analysing the amplified product by comparison with the molecule of sequence SEQ ID NO:21.

4. Method of detection according to claim 3, **characterised in that** the analysis step (3) can be carried out by restriction, sequencing or hybridisation.

5. Method according to claim 3 or claim 4, **characterised in that** the specific primers for amplification of the R0, R1, R2, R3, R644, G910, p910, R'1 and R'2 regions used in step (2) are selected from the group consisting of:
- the primers 900S (SEQ ID NO:33) and 1280AS (SEQ ID NO:34), for the amplification of R0 (approximately 400 pb),
- the primers 320S (SEQ ID NO:39) and 1280AS (SEQ ID NO:34), for the amplification of the R1 fragment (approximately 960 pb),
- the primers 900S (SEQ ID NO:33) and 320AS (SEQ ID NO:45), for the amplification of R2 (approximately 1100 pb), which contains the sHD gene corresponding to positions 1598 to 950,
- the primers 1480S (SEQ ID NO:46) and 440AS (SEQ ID NO:47), for the amplification of R3 (approximately 650 pb),
- the primers 318S (SEQ ID NO:35) and 1150AS (SEQ ID NO:36), for the amplification of R'1 (approximately 850 pb),
- the primers 960S (SEQ ID NO:37) and 345AS (SEQ ID NO:38), for the amplification of R'2 (approximately 1050 pb).

6. Method of detection and of genotyping of HDV from a biological sample, which method is **characterised in that** it comprises:
(a) a step of extracting the nucleic acid belonging to the genome of the HDV virus,
(b) a step of amplifying the R0 region delimited by positions 889 to 1289 of the HDV genome,
(c) a first treatment of the amplified nucleic acid molecules with the *Sma*I and *Xho*I restriction enzymes to produce a first set of restriction fragments,
(d) a second treatment of the nucleic acid molecules with the SacII restriction enzyme to produce a second set of restriction fragments,
(e) the combined analysis of the two sets of restriction fragments produced by RFLP (*restriction fragment length polymorphism*), so as to detect the presence of dFr48 HDV present in said biological sample.

7. Method according to claim 6, **characterised in that** step (b) comprising amplification of the nucleic acid molecules of said sample by RT-PCR is advantageously performed with the primers 900S (SEQ ID NO:33) and 1280AS (SEQ ID NO:34).

8. Method according to claim 6 or claim 7, **characterised in that** it also comprises:
(f) amplification of the nucleic acid molecules of said sample by RT-PCR with the primers 900S (SEQ ID NO:33) and 320AS (SEQ ID NO:45), so as to amplify the R2 region, and
(g) direct sequencing of the amplified R2 region and comparison with the RNA molecule of sequence SEQ ID NO:21.

9. Recombinant vector, in particular a plasmid, comprising an insert consisting of a nucleic acid molecule according to one of claims 1 or 2.

10. Cell transformed by a nucleic acid molecule according to one of claims 1 or 2.

11. Translation products encoded by the RNA molecule of sequence SEQ ID NO:21, by its complementary sense or antisense sequences.

12. Proteins **characterised in that** they are encoded by one of the nucleic acid molecule according to one of claims 1 or 2.

13. Protein according to claim 12, **characterised in that** it is selected from the group consisting of:
- the LHD protein of dFr48, which has the sequence SEQ ID NO:23, and
- the sHD protein of dFr48 which has the sequence SEQ ID NO:25.

14. Peptide **characterised in that** it consists of a fragment of a protein according to claim 12 or claim 13, selected from the group consisting of:
- peptide A consisting of the 19 amino acids of the carboxy-terminal end of the sequence SEQ ID NO:23,
- peptide C of sequence SEQ ID NO:64.

15. Use of a nucleic acid molecule according to one of claims 1 or 3 or of a protein according to one of claims 11 to 14 to prepare an HDV detection and genotyping kit.

## Patentansprüche

1. Isolierte Nukleinsäuremoleküle, **dadurch gekennzeichnet, dass** sie ausgewählt sind aus der Gruppe bestehend aus:
- dem gesamten Genom der HDV-Variantc mit der Bezeichnung dFr45, die die Sequenz SEQ-ID Nr. 21 aufweist, und
- dem Genom einer HDV-Variante, die eine genetische Divergenz von ≤ 15% zu der Sequenz SEQ-ID Nr. 21 aufweist.

2. Isolierte Nukleinsäuremoleküle, **dadurch gekennzeichnet, dass** sie wenigstens eines der Fragment der Sequenzen einer HDV-Variante nach Anspruch 1 umfassen, ausgewählt aus der Gruppe bestehend aus:
a) dem Fragment R0 mit der SEQ-ID Nr. 50,
b) dem Fragment R1, das sich über die Positionen 307 bis 1289 des HDV-Genoms erstreckt,
c) dem Fragment R2, das sich über die Positionen 889 bis 328 des HDV-Genoms erstreckt,
d) dem Fragment R3, das sich über die Positionen 1486 bis 452 des HDV-Genoms erstreckt,
e) dem Fragment R'1, das sich über die Positionen 305 bis 1161 des HDV-Genoms erstreckt,
f) dem Fragment R'2, das sich über die Positionen 984 bis 331 des HDV-Genoms erstreckt,
g) dem Fragment R644, das sich über die Positionen 889 bis 446 des HDV-Genoms erstreckt,
h) dem Fragment G910, das sich über die Positionen 1206 bis 929 des HDV-Genoms erstreckt,
i) dem Fragment p910, das sich über die Positionen 553 bis 1550 des HDV-Genoms erstreckt,
j) der cDNA der Sequenz SEQ-ID Nr. 24, die für das sHD-Protein codiert,
k) der cDNA der Sequenz SEQ-ID Nr. 22, die für das LDH-Protein codiert.

3. Verfahren zum Detektieren einer HDV-Variante gemäß der Definition in Anspruch 1 durch Hybridisierung und/oder Amplifizierung, das ausgehend von einer biologischen Probe umgesetzt wird, wobei das Verfahren **dadurch gekennzeichnet ist, dass** es folgendes umfasst:
(1) einen Schritt der Extraktion der zu detektierenden Nukleinsäure, die zu dem Genom des Virus gehört, das möglicherweise in der biologischen Probe vorliegt,
(2) Umsetzung wenigstens einer genetischen Amplifizierung mithilfe eines Primerpaares, das ausgewählt ist aus der Gruppe bestehend aus den Primern, die geeignet sind, eine der folgenden Regionen der genomischen RNA des HDV zu amplifizieren: R0, R1, R2, R3, R644, G910, p910, R'1 und R'2, und
(3) Analyse des amplifizierten Produkts durch Vergleich mit dem Molekül der Sequenz SEQ-ID Nr. 21.

4. Verfahren zum Detektieren nach Anspruch 3, **dadurch gekennzeichnet, dass** der Schritt (3) der Analyse durch Restriktion, Sequenzierung oder Hybridisierung durchgeführt werden kann.

5. Verfahren nach Anspruch 3 oder Anspruch 4, **dadurch gekennzeichnet, dass** die spezifischen Primer für die Amplifizierung der Regionen R0, R1, R2, R3, R644, G910, p910, R'1 und R'2, die in Schritt (2) verwendet werden, ausgewählt sind aus der Gruppe bestehend aus:
- den Primern 900S (SEQ-ID Nr. 33) und 1280AS (SEQ-ID Nr. 34) zum Amplifizieren von R0 (ca. 400 bp),
- den Primern 320S (SEQ-ID Nr. 39) und 1280AS (SEQ-ID Nr. 34) zum Amplifizieren des Fragments R1 (ca. 960 bp),
- den Primern 900S (SEQ-ID Nr. 33) und 320AS (SEQ-ID Nr. 45) zum Amplifizieren von R2 (ca. 1100 bp), welches das den Positionen 1598 bis 950 entsprechende sHD-Gen enthält,
- den Primern 1480S (SEQ-ID Nr. 46) und 440AS (SEQ-ID Nr. 47) zum Amplifizieren von R3 (ca. 650 bp),
- den Primern 318S (SEQ-ID Nr. 35) und 1150AS (SEQ-ID Nr. 36) zum Amplifizieren von R'1 (ca. 850 bp),
- den Primern 960S (SEQ-ID Nr. 37) und 345AS (SEQ-ID Nr. 38) zum Amplifizieren von R'2 (ca. 1050 bp).

6. Verfahren zum Detektieren und Genotypisieren von HDV ausgehend von einer biologischen Probe, wobei das Verfahren **dadurch gekennzeichnet ist, dass** es folgendes umfasst:
(a) einen Schritt der Extraktion der Nukleinsäure, die zu dem Genom des HDV-Virus gehört,
(b) einen Schritt der Amplifizierung der Region R0, die von den Positionen 889 bis 1289 des HDV-Genoms begrenzt wird,
(c) eine erste Behandlung der mit den Restriktionsenzymen *Sma*I und *Xho*I amplifizierten Nukleinsäuremoleküle, um eine erste Gesamtheit von Restriktionsfragmenten herzustellen,
(d) eine zweite Behandlung der Nukleinsäuremoleküle mit dem Restriktionsenzym *Sac*II, um eine zweite Gesamtheit von Restriktionsfiaginenten herzustellen,
(e) eine Kombinationsanalyse der beiden Gesamtheiten von durch RFLP (*Restriction Fragment Length Polymorphism*) hergestellten Restriktionsfragmenten, derart, dass die Anwesenheit des in der biologischen Probe vorliegenden HDV dFr48 detektiert wird.

7. Verfahren nach Anspruch 6, **dadurch gekennzeichnet, dass** der Schritt (b) des Amplifizierens der Nukleinsäuremoleküle der Probe durch RT-PCR vorteilhafterweise mit den Primern 900S (SEQ-ID Nr. 33) und 1280AS (SEQ-ID Nr. 34) durchgeführt wird.

8. Verfahren nach Anspruch 6 oder Anspruch 7, **dadurch gekennzeichnet, dass** es ferner folgendes umfasst:
(f) Amplifizieren der Nukleinsäuremoleküle der Probe durch RT-PCR mit den Primern 900S (SEQ-ID Nr. 33) und 320AS (SEQ-ID Nr. 45), derart, dass die Region R2 amplifiziert wird, und
(g) direktes Sequenzieren der amplifizierten Region R2 und Vergleich mit dem RNA-Molekül der Sequenz SEQ-ID Nr. 21.

9. Rekombinanter Vektor, insbesondere ein Plasmid, umfassend ein aus einem Nukleinsäuremolekül nach einem der Ansprüche 1 oder 2 bestehendes Insert.

10. Zelle, die mit einem Nukleinsäuremolekül nach Anspruch 1 oder 2 transformiert ist.

11. Translationsprodukte, die von dem RNA-Molekül der Sequenz SEQ-ID Nr. 21 oder von dessen komplementärer Sense- oder Antisense-Sequenz codiert werden.

12. Proteine, **dadurch gekennzeichnet, dass** sie von einem der Nukleinsäuremoleküle der Ansprüche 1 oder 2 codiert werden.

13. Protein nach Anspruch 12, **dadurch gekennzeichnet, dass** es ausgewählt ist aus der Gruppe bestehend aus:
- dem LDH-Protein von dFr48, das die Sequenz SEQ-ID Nr. 23 aufweist,
- dem sHD-Protein von dFr48, das die Sequenz SEQ-ID Nr. 25 aufweist.

14. Peptid, **dadurch gekennzeichnet, dass** es von einem Fragment eines Proteins nach Anspruch 12 oder Anspruch 13 gebildet ist, ausgewählt aus der Gruppe bestehend aus:
- dem Peptid A, das aus den 19 Aminosäuren des Carboxy-terminalen Endes der Sequenz SEQ-ID Nr. 23 gebildet ist,
- dem Peptid C der Sequenz SEQ-ID Nr. 64.

15. Verwendung eines Nukleinsäuremoleküls nach einem der Ansprüche 1 oder 3 oder eines Proteins nach einem der Ansprüche 11 bis 14 zum Herstellen eines Kits zur Detektion und Genotypisierung eines HDV.
